(19) **Europäisches Patentamt**
**European Patent Office**
**Office européen des brevets**

(11) **EP 3 867 913 B1**

(12) **EUROPÄISCHE PATENTSCHRIFT**

(45) Veröffentlichungstag und Bekanntmachung des Hinweises auf die Patenterteilung:
**28.01.2026 Patentblatt 2026/05**

(21) Anmeldenummer: **19784110.9**

(22) Anmeldetag: **16.10.2019**

(51) Internationale Patentklassifikation (IPC):
**G16C 20/10** (2019.01)

(52) Gemeinsame Patentklassifikation (CPC):
**G16C 20/10**

(86) Internationale Anmeldenummer:
**PCT/EP2019/078111**

(87) Internationale Veröffentlichungsnummer:
**WO 2020/079093 (23.04.2020 Gazette 2020/17)**

(54) **MONTE-CARLO-METHODE ZUR AUTOMATISIERTEN UND HOCH-EFFIZIENTEN BERECHNUNG VON KINETISCHEN DATEN CHEMISCHER REAKTIONEN**

MONTE CARLO METHOD FOR THE AUTOMATED AND HIGHLY EFFICIENT CALCULATION OF KINETIC DATA OF CHEMICAL REACTIONS

MÉTHODE DE MONTE CARLO POUR LE CALCUL AUTOMATISÉ ET HAUTEMENT EFFICACE DE DONNÉES CINÉTIQUES DE RÉACTIONS CHIMIQUES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **18.10.2018 EP 18201340**

(43) Veröffentlichungstag der Anmeldung:
**25.08.2021 Patentblatt 2021/34**

(73) Patentinhaber: **Covestro Deutschland AG**
**51373 Leverkusen (DE)**

(72) Erfinder:
• **GAMEZ, Jose**
**51063 Köln (DE)**
• **LEVEN, Matthias**
**51069 Köln (DE)**

(74) Vertreter: **Levpat**
**c/o Covestro AG**
**Gebäude 4825**
**51365 Leverkusen (DE)**

(56) Entgegenhaltungen:
EP-A1- 2 352 107        WO-A1-2018/102565
CN-A- 104 765 918       JP-A- 2008 250 392
JP-A- H1 125 063        US-A1- 2003 236 655

• LIN X.-X. ET AL: "A flexible transition state searching method for atmospheric reaction systems", CHEMICAL PHYSICS, NORTH-HOLLAND, NL, vol. 450, 11 February 2015 (2015-02-11), pages 21 - 31, XP029204426, ISSN: 0301-0104, DOI: 10.1016/ J.CHEMPHYS.2015.02.002
• SWIHART M. ET AL: "Assembling gas-phase reaction mechanisms for high temperature inorganic systems based on quantum chemistry calculations and reaction rate theories", JOURNAL OF PHYSICS AND CHEMISTRY OF SOLIDS, PERGAMON PRESS, LONDON, GB, vol. 66, no. 2-4, 1 February 2005 (2005-02-01), pages 364 - 371, XP027709615, ISSN: 0022-3697, [retrieved on 20050201]
• HAFNER J.: "Adsorption and reaction of organic molecules on solid surfaces - ab-initio density functional investigations", MONATSHEFTE FÜR CHEMIE - CHEMICAL MONTHLY ; AN INTERNATIONAL JOURNAL OF CHEMISTRY, SPRINGER-VERLAG, AU, vol. 139, no. 4, 14 March 2008 (2008-03-14), pages 373 - 387, XP019592602, ISSN: 1434-4475

- MARTÍNEZ-NÚÑEZ E.: "An automated transition state search using classical trajectories initialized at multiple minima", PHYSICAL CHEMISTRY CHEMICAL PHYSICS, vol. 17, no. 22, 6 May 2015 (2015-05-06), pages 14912 - 14921, XP055601422, ISSN: 1463-9076, DOI: 10.1039/C5CP02175H
- RODRÍGUEZ A. ET AL: "tsscds2018: A code for automated discovery of chemical reaction mechanisms and solving the kinetics", JOURNAL OF COMPUTATIONAL CHEMISTRY., vol. 39, no. 23, 5 September 2018 (2018-09-05), GB, pages 1922 - 1930, XP055601919, ISSN: 0192-8651, DOI: 10.1002/jcc.25370
- JACOBSON L. D.ET AL: "Automated Transition State Search and Its Application to Diverse Types of Organic Reactions", JOURNAL OF CHEMICAL THEORY AND COMPUTATION: JCTC, vol. 13, no. 11, 17 October 2017 (2017-10-17), US, pages 5780 - 5797, XP055601412, ISSN: 1549-9618, DOI: 10.1021/acs.jctc.7b00764
- SALCICCIOLI M. ET AL: "A review of multiscale modeling of metal-catalyzed reactions: Mechanism development for complexity and emergent behavior", CHEMICAL ENGINEERING SCIENCE, OXFORD, GB, vol. 66, no. 19, 30 May 2011 (2011-05-30), pages 4319 - 4355, XP028264661, ISSN: 0009-2509, [retrieved on 20110613], DOI: 10.1016/J.CES.2011.05.050
- KEIL F. J.: "Complexities in modeling of heterogeneous catalytic reactions", COMPUTERS & MATHEMATICS WITH APPLICATIONS, ELSEVIER, AMSTERDAM, NL, vol. 65, no. 10, 3 January 2013 (2013-01-03), pages 1674 - 1697, XP028556589, ISSN: 0898-1221, DOI: 10.1016/J.CAMWA.2012.11.023
- DIEDRICH M. K. ET AL: "Experimental Determination of the Activation Parameters and Stereoselectivities of the Intramolecular Diels-Alder Reactions of 1,3,8-Nonatriene, 1,3,9-Decatriene, and 1,3,10-Undecatriene and Transition State Modeling with the Monte Carlo-Jumping Between Wells/Molecular Dynamics Method", JOURNAL OF THE AMERICAN CHEMICAL SOCIETY, vol. 119, no. 43, 1 October 1997 (1997-10-01), pages 10255 - 10259, XP055593729, ISSN: 0002-7863, DOI: 10.1021/ja9643331
- MERCERO J. M. ET AL: "Theoretical methods that help understanding the structure and reactivity of gas phase ions", INTERNATIONAL JOURNAL OF MASS SPECTROMETRY, ELSEVIER SCIENCE PUBLISHERS, AMSTERDAM, NL, vol. 240, no. 1, 1 January 2005 (2005-01-01), pages 37 - 99, XP027705154, ISSN: 1387-3806, [retrieved on 20050101]
- KRATZER P.: "Monte Carlo and kinetic Monte Carlo methods", ARXIV.ORG, CORNELL UNIVERSITY LIBRARY, 201 OLIN LIBRARY CORNELL UNIVERSITY ITHACA, NY 14853, 16 April 2009 (2009-04-16), XP080320087
- GREBNER C. ET AL: "PathOpt-A global transition state search approach: Outline of algorithm", JOURNAL OF COMPUTATIONAL CHEMISTRY., vol. 34, no. 21, 4 May 2013 (2013-05-04), GB, pages 1810 - 1818, XP055601427, ISSN: 0192-8651, DOI: 10.1002/jcc.23307
- LIN Y. ET AL: "Reliable Modeling and Optimization for Chemical Engineering Applications: Interval Analysis Approach", RELIABLE COMPUTING ; AN INTERNATIONAL JOURNAL DEVOTED TO RELIABLE MATHEMATICAL COMPUTATIONS BASED ON FINITE REPRESENTATIONS AND GUARANTEED ACCURACY, KLUWER ACADEMIC PUBLISHERS, DO, vol. 12, no. 6, 25 October 2006 (2006-10-25), pages 427 - 450, XP019453859, ISSN: 1573-1340, DOI: 10.1007/S11155-006-9013-6

**Beschreibung**

Die vorliegende Erfindung betrifft ein computerimplementiertes Verfahren zur Berechnung von Übergangszuständen einer chemischen Reaktion, sowie ein System zur Datenverarbeitung umfassend Mittel zur Ausführung des Verfahrens, ein Computerprogramm umfassend Befehle die einen Computer veranlassen das Verfahren durchzuführen und die Verwendung des Computerprogramms. Die Erfindung betrifft außerdem ein System, ein Verfahren und ein Mittel für die automatisierte und effiziente Bestimmung von kinetischen Daten chemischer Reaktionen.

Während einer chemischen Reaktion verändern die beteiligten Atome ihre Geometrie und Bindungen werden gebrochen und neue Bindungen gebildet. Dabei verändert sich auch die Energie der beteiligten Atome und erreicht während des Verlaufs der chemischen Reaktion einen Zustand maximaler Energie, den sogenannten Übergangszustand. Der Übergangszustand stellt einen Potentialwall oder eine Aktivierungsbarriere dar, der die Edukte von den Produkten der chemischen Reaktion trennt. Ist die Aktivierungsbarriere überwunden, so wird das Produkt gebildet. Die Energie während einer chemischen Reaktion kann mit Hilfe einer sogenannten Potentialhyperfläche dargestellt werden, welche die potentielle Energie der an der Reaktion beteiligten Atome in Abhängigkeit von ihrer Geometrie abbildet. Dabei bildet die Potentialhyperfläche auch Zustände ab, bei denen keine Produkte gebildet werden. Der direkte Reaktionspfad, bei dem Produkte unter Überwindung des Übergangszustandes gebildet werden, kann als Kurve gezeigt werden, bei der die Abstände der einzelnen Atome der Moleküle gegen die Energie aufgetragen werden. Mit Hilfe von quantenchemischen Methoden und mathematischen Näherungsverfahren kann die Energie der Geometrie eines Moleküls oder eines Systems von mehreren Molekülen berechnet werden, wobei der dabei zu betrachtende Funktionsraum die Freiheitsgrade des Moleküls umfasst. Diese Verfahren ermöglichen die Ermittlung der Geometrie am Übergangszustand und erlauben Vorhersagen über die Reaktionskinetik einer chemischen Reaktion.

Eine bekannte Methode mit der die Geometrie am Übergangszustand ermittelt werden kann, sind Quasi-Newton-Raphson-Verfahren, auch Pseudo-Newton-Raphson Verfahren genannt. Die Berechnung von Übergangszuständen mit Hilfe von Quasi-Newton-Raphson-Verfahren weist jedoch einige Nachteile auf. Zunächst führen Quasi-Newton-Raphson Algorithmen nicht bei jeder beliebigen Ausgangsgeometrie eines Molekül zu einem Übergangszustand sondern nur bei Molekülgeometrien, die bereits sehr nah an die Geometrie des Übergangszustandes angenähert sind. Die Annäherung ist aufwendig, weil die Annäherung einer Molekülgeometrie an die Geometrie des Übergangszustandes üblicherweise händisch erfolgt, d.h. es werden Bindungslängen per Hand am Computer eingestellt. Es sind auch Methoden bekannt, bei denen zunächst mit anderen Methoden Potentialhyperflächen berechnet werden aus denen mögliche Sattelpunkte identifiziert werden. Auch bei diesen Methoden muss also erst eine Geometrie ermittelt werden, die bereits sehr nah an die Geometrie des Übergangszustandes angenähert ist. Des Weiteren sind Quasi-Newton-Raphson Verfahren anfällig für Fehler wenn sie auf derartige, händisch ausgewählte Molekülgeometrien angewendet werden. Pseudo-Newton-Raphson basierte Verfahren führen oft dazu, dass an Stelle der benötigten Sattelpunkte erster Ordnung lokale Minima erhalten werden. Das liegt daran, dass in der Theorie der Newton-Raphson basierten Methoden der Übergangszustand selbst in einer Potenzreihe an der Stelle Gradient=0 entwickelt wird. Ist die händisch generierte Geometrie zu weit vom Übergangszustand selbst entfernt, ist die Voraussetzung Gradient≈0 nicht ausreichend erfüllt um eine Konvergenz zu erreichen. Außerdem wird in Quasi-Newton-Raphson Verfahren die Hessematrix nur im ersten Schritt berechnet. In den folgenden Schritten wird diese durch Update-Algorithmen geschätzt, was eine Fehlerquelle darstellt. Je weiter die Anfangsstruktur vom Übergangszustand entfernt ist, umso mehr Iterationen bzw. Updates werden benötigt, desto größer ist der Fehler oder Abstand zur richtigen Berechnung der Hessematrix, und desto weiter ist der Abstand zu einer Konvergenz.

Lin et al. ("A flexible transition state searching method for atmospheric reaction systems," Chemical Physics 450-451, 2015, S. 21-31) offenbaren eine Methode zur Untersuchung atmosphärischer chemischer Reaktionen in der Gasphase. Die Methode umfasst in einem ersten Schritt ein auf Monte-Carlo-Methoden basiertes Screening von Potentialhyperflächen mit Kraftfeld-Methoden, wobei genäherte Sattelpunkt-artige Regionen identifiziert werden. Die verwendete Observable der entsprechenden Monte Carlo Methode ist dabei der Wert einer Energiefunktion. Darauf aufbauend wird dann versucht mittels einer Quasi Newton-Raphson Methode-chemische Übergangszustände zu lokalisieren. Mit der hier offenbarten Monte-Carlo-Methode muss zunächst die gesamte Potentialhyperfläche simuliert werden, bevor überhaupt genäherte Sattelpunkt-artige Regionen identifiziert werden können. Folglich können mit dieser Monte-Carlo-Methode nicht gezielt Sattelpunkt-artige Regionen optimiert werden. Aus diesem Grund ist keine effiziente Nutzung der verwendeten Rechenressourcen zur Lokalisierung der benötigten Sattelpunkte möglich. Der Einsatz der hier offenbarten Methode beschränkt sich mit der derzeitigen Computertechnik auf kleine Moleküle mit bis zu 30 Atomen. Zudem werden mathematisch stark vereinfachte Methoden verwendet.

E. Martínez-Núñez et al. "An automated transition state search using classical trajectories initialized at multiple minima", Phys Chem Chem Phys, 14. Juni 2015, 17(22):14912-21; "tsscds2018: A code for automated discovery of chemical reaction mechanisms and solving the kinetics", J. Comp.Chem., 24. September 2018, 39(23)1922-1930) offenbaren eine Methode zur Untersuchung chemische Reaktionspfade. Dabei werden Potentialhyperflächen chemischer Moleküle mit mathematisch vereinfachten Methoden berechnet. Anschließend wird versucht die chemisch relevanten Übergangszustände mit gewöhnlichen Pseudo-Newton-Raphson-Methoden zu berechnen. Auch hier ist nachteilig, dass zunächst die gesamte Potentialhyperfläche berechnet werden muss, was zeitaufwändig ist und Rechnerleistung braucht. Zudem kann auch diese Methode nur auf kleinere Moleküle angewendet werden.

Jacobson et al. ("Automated Transition State Search and Its Application to Diverse Types of Organic Reactions", J. Chem. Theory Comput. 13, 11, 5780-5797) offenbaren eine Methode zur automatisierten Berechnung von chemischen Übergangszuständen. Dabei werden chemische Übergangszustände aus chemischen Gleichgewichtszuständen berechnet. Dieses Vorgehen benötigt eine separate Berechnung von chemischen Gleichgewichtszuständen, bevor eine Interpolation der erhaltenen Geometrien durchgeführt werden kann, mit welchen der Übergangszustand angenähert wird. In einem weiteren Schritt wird dann basierend auf dieser Annäherung versucht eine Übergangszustandsgeometrie zu berechnen. Das Erstellen der benötigten Gleichgewichtszustände erfordert zusätzlichen Arbeitsaufwand durch den Fachmann. Die in der offenbarten Methode verwendete Interpolation lässt sich nicht mit Erfolg auf beliebige Molekülgeometrien anwenden.

Hu et al. ("A gradient-directed Monte Carlo method for global optimization in a discrete space: Application to protein sequence design and folding" J Chem Phys. 2009 Oct 21; 131(15): 154117) offenbaren eine Methode zur Berechnung von Proteinstrukturen offenbart. Bei dieser Methode werden Faltungen von Proteinstrukturen mittels Monte-Carlo-Methoden berechnet. Zur Beschleunigung der Konvergenz der Monte-Carlo-Prozeduren werden Gradienten berechnet, welche in die Richtung und Größe von Auslenkungen der Atompositionen eingehen. Die Publikation lässt keine mögliche Verwendung dieser Methode zur gezielten Berechnung von Sattelpunkten erkennen.

Nachteilig an den bekannten Verfahren zur Berechnung von Übergangständen ist die Kombination unterschiedlicher quantenchemischer Verfahren zur Berechnung einer Potentialhyperfläche oder einer Ausgangsgeometrie einerseits und zur Berechnung des Sattelpunktes andererseits. Die Potentialhyperfläche, die mit einem quantenchemischen Verfahren berechnet wird, ist nicht unbedingt geeignet ist den Sattelpunkt mit einem anderen quantenchemischen Verfahren anderer Qualität zu berechnen, da unterschiedliche quantenchemische Methoden Parameter bei der Näherung der Potentialhyperfläche unterschiedliche Genauigkeiten ergeben. Daher sind die mit diesen Verfahren erhaltenen Sattelpunkte häufig ungenau oder sogar weit vom tatsächlichen Sattelpunkt entfernt. Des Weiteren ist die händische Annäherung einer Molekülgeometrie an die Geometrie des Übergangszustandes zeit- und personalaufwendig.

Es besteht somit ein Bedarf für ein Verfahren für die Berechnung von Übergangszuständen, bei dem eine Molekülgeometrie für eine Übergangszustands einer chemischen Reaktion mit einem einfachen Verfahren, insbesondere einem computerimplementierten Verfahren, genähert werden kann, bevor mit einer Berechnung des Übergangszustands mit Hilfe eines quantenchemischen Verfahrens, insbesondere eines Pseudo-Newton-Raphson-Verfahrens, begonnen werden kann. Insbesondere besteht ein Bedarf für ein Verfahren mit dem Übergangszustände ermittelt werden können, ohne zuvor eine Potentialhyperfläche berechnen zu müssen. Hierbei werden Verfahren verwendet die großen Rechenaufwand mit sich bringen und qualitativ weniger genau sind. Auf diese Weise können Auslastungen von Ressourcen wie Prozessoren und Speichermedien reduziert werden.

Aufgabe der vorliegenden Erfindung war ein computerimplementiertes Verfahren zur Verfügung zu stellen, bei dem eine Geometrie eines Übergangszustandes mit einem leicht anwendbaren, insbesondere einem computerimplementierten, Verfahren angenähert werden kann, bevor in einem folgenden Schritt der Übergangszustand mit Hilfe von quantenchemischen Verfahren, insbesondere von Pseudo-Newton-Raphson-Algorithmen, berechnet wird. Insbesondere soll eine Molekülgeometrie für einen Übergangszustand einer chemischen Reaktion ermittelt werden können, ohne zuvor die Potentialhyperfläche der chemischen Reaktion zu berechnen.

Diese Aufgabe wurde gelöst durch ein computerimplementiertes Verfahren zur Berechnung von Übergangszuständen einer chemischen Reaktion umfassend die Schritte

**A Erzeugen einer Startgeometrie**

A1 Bereitstellung der dreidimensionalen Darstellung mindestens eines Moleküls im energetischen Grundzustand,
A2 Auswahl von mindestens einer Bindung des mindestens einen Moleküls und Auswahl einer Länge der Bindung, wobei die ausgewählte Länge nicht der Länge der Bindung im energetischen Grundzustand des Moleküls entspricht, so dass eine Startgeometrie für die chemische Reaktion erhalten wird,
A3 Dreidimensionale Darstellung der Startgeometrie in kartesischen , iund/oder internen Koordinaten,

**B Ermittlung einer optimierten Startgeometrie**

B1 Festlegung eines Funktionsraumes welcher die mindestens eine Bindung aus Schritt A2 und die durch diese Bindung verbundenen Atome umfasst,

B2 Geometrieoptimierung der Startgeometrie unter Zugrundelegung des in Schritt B1 ausgewählten Funktionsraums mittels einer quantenchemischen Methode und mit der Randbedingung, dass die in Schritt A2 ausgewählte Länge der mindestens einen Bindung konstant gehalten wird, so dass eine optimierte Startgeometrie erhalten wird,

B3 Ermittlung der Gradient-Norm B3 für die optimierte Startgeometrie, wobei die Gradient-Norm durch die erste Ableitung einer Funktion E = f(x) mittels der quantenchemischen Methode erhalten wird, mit E=Gesamtenergie der optimierten Startgeometrie und x=Kernkoordinaten des Moleküls in der optimierten Startgeometrie,

B4.1 wenn die Gradient-Norm B3 $\nabla$ $0 \le \nabla \le 0{,}07$ $E_h\,a_0^{-1}$ beträgt, dann Einordnung der optimierten Startgeometrie als eine Vorstufe für den Übergangszustand der chemischen Reaktion und Fortführung des Verfahrens mit Schritt D1, oder

B4.2 wenn die Gradient-Norm B3 $\nabla > 0{,}07$ $E_h\,a_0^{-1}$ beträgt, dann Ermittlung der Vorstufe für den Übergangszustand der chemischen Reaktion ausgehend von der optimierten Startgeometrie durch ein Verfahren umfassend die folgenden Schritte:

## C Ermittlung der Vorstufe für den Übergangszustand der chemischen Reaktion

C1 Variation der optimierten Startgeometrie unter Anwendung eines Monte-Carlo-Algorithmus wobei

C1.1 wenigstens ein Atom aus dem in Schritt B1 ausgewählten Funktionsraum zufällig ausgewählt wird,

C1.2 ein Vektor für eine Auslenkung des in Schritt C1.1 gewählten Atoms zufällig ausgewählt wird,

C1.3 das in Schritt C1.1 ausgewählte Atom anhand des Vektors aus Schritt C1.2 aus seiner Position in der optimierten Startgeometrie ausgelenkt wird, so dass eine Vorstufe für den Übergangszustand der chemischen Reaktion erhalten wird,

C2 Geometrieoptimierung der Vorstufe für den Übergangszustand mittels der quantenchemischen Methode und mit der Randbedingung, dass die mindestens eine Bindung aus Schritt A2 die Länge hat, die in der Schritt C1.3 ermittelt wurde,

C3 Ermittlung der Gradient-Norm C3 für die Vorstufe für den Übergangszustand aus Schritt C2, wobei die Gradient-Norm durch die erste Ableitung der Funktion E = f(x) mittels der quantenchemischen Methode erhalten wird, mit E=Gesamtenergie der Vorstufe für den Übergangszustand und x=Kernkoordinaten des Moleküls in der Vorstufe für den Übergangszustand,

C4.1 wenn die Gradient-Norm C3 $\nabla$ $0 \le \nabla \le 0{,}07$ $E_h\,a_0^{-1}$ beträgt, dann Fortführung des Verfahrens mit Schritt D1, oder

C4.2 wenn die Gradient-Norm C3 $\nabla > 0{,}07$ $E_h\,a_0^{-1}$ beträgt, dann Wiederholung der Schritte C1 bis C3 bis eine Gradient-Norm C3 $0 \le \nabla \le 0{,}07$ $E_h\,a_0^{-1}$ erhalten wird, wobei

(a) für den Fall, dass die Schritte C1 bis C3 ein Mal durchgeführt wurden, in Schritt C1 die Geometrie der Vorstufe für den Übergangszustand variiert wird, wenn der Wert ihrer Gradient-Norm C3 niedriger ist als der Wert der Gradient-Norm B3 der optimierten Startgeometrie, oder

(b) für den Fall dass die Schritte C1 bis C3 mehr als ein Mal durchgeführt wurden, in Schritt C1 die Geometrie der optimierten Startgeometrie oder derjenigen Vorstufe für den Übergangszustand aus den vorrangegangen Wiederholungen variiert wird, welche im Vergleich zu allen bisher erhaltenen Gradient-Normen C3 und B3 den niedrigsten Wert für die Gradient-Norm C3 oder B3 aufweist,

## D Ermittlung des Übergangszustandes

D1 Relaxation der Vorstufe aus Schritt C4.1 oder der Vorstufe aus Schritt B4.1 mittels der quantenchemischen Methode und eines Pseudo-Newton-Raphson-Algorithmus, so dass der Übergangszustand erhalten wird,

D2 gegebenenfalls Ermittlung eines Gleichgewichtszustands durch Auslenkung des Übergangszustands, so dass ein ausgelenkter Übergangszustand erhalten wird und Relaxation des ausgelenkten Übergangszustands mittels der quantenchemischen Methode, so dass ein Gleichgewichtszustand erhalten wird.

Es wurde überraschend gefunden, dass die Aufgabe gelöst werden kann, indem durch Einsatz einer geeigneten Monte-Carlo-Methode verschiedene Molekülgeometrien variiert werden und mit Hilfe eines Gütekriteriums eine Geometrie für einen Übergangszustand angenähert wird. Des Weiteren wurde überraschend gefunden, dass die Aufgabe gelöst werden kann, indem bei dem Monte-Carlo-verfahren als Observable der Gradient-Norm der Kurve des direkten Reaktionspfades verwendet wird. Dadurch lässt sich der zu behandelnde Funktionsraum, der die Molekülgeometrie am Übergangszustand der chemischen Reaktion abbilden soll, derart reduzieren, dass die Berechnung von Sattelpunkten ermöglicht wird, ohne zuvor eine Potentialhyperfläche zu berechnen oder manuell Molekülgeometrien zu suchen. Außerdem wurde überraschend gefunden, dass mit dem erfindungsgemäßen Verfahren die Berechnung von Sattelpunkten in hochdimensionalen Funktionsräumen möglich wird. Ein weiterer Vorteil des erfindungsgemäßen Verfahrens ist, dass die quantenchemischen Berechnungen bei der Durchführung des Verfahrens mit der gleichen Methode durchgeführt werden. Mit der derzeitigen Computertechnik können mit dem erfindungsgemäßen Verfahren deutlich kürzer und schneller auch Übergangszustände für Moleküle mit bis zu 100 Atomen berechnet werden, vor allem auch durch die Reduktion von personellem Aufwand der zur Bearbeitung entsprechender herkömmlicherProzesse notwendig ist. Hierbei werden auch Computer Ressourcen gespart.

Im erfindungsgemäßen Verfahren wird in **Schritt A** des wird zunächst eine Startgeometrie erzeugt, indem in Schritt A1 die dreidimensionale Darstellung mindestens eines Moleküls im energetischen Grundzustand bereitgestellt wird. Dann wird in Schritt A2 eine Bindung des in Schritt A1 dreidimensional dargestellten Moleküls ausgewählt und eine Länge der Bindung, die von der Länge der Bindung im energetischen Grundzustand abweicht, so dass eine Startgeometrie erhalten wird. Bei den ausgewählten Bindungen handelt es sich bevorzugt um diejenigen Bindungen welche in der zu betrachtenden chemischen Reaktion gebildet oder gebrochen werden. Die Bindungslänge, die von der Länge der Bindung im energetischen Grundzustand abweicht ist um 10 bis 90% bevorzugt 20% bis 40 % größer als die Bindung im energetischen Grundzustand.

Bevorzugt weist das mindestens eine Molekül aus Schritt A1 eine Größe von höchstens 100 Atomen auf, weiter bevorzugt von höchstens 80 Atomen, bevorzugter von höchstens 60 Atomen und/oder beträgt die Länge der in Schritt **A2** ausgewählten Bindung höchstens 230 pm, bevorzugt höchstens 200 pm, weiter bevorzugt höchstens 180 pm, bevorzugter 150 pm.

In einer weiteren bevorzugten Ausführungsform des erfindungsgemäßen Verfahrens werden in Schritt A1 mindestens zwei Moleküle I und II bereitgestellt und in Schritt **A2** können alternativ oder zusätzlich zu der mindestens einen Bindung auch mindestens ein Abstand zwischen mindestens einem Atom aus Molekül I und mindestens einem Atom aus Molekül II sowie die Länge des mindestens einen Abstands ausgewählt werden, wobei die Länge des Abstands insbesondere höchstens 230 pm beträgt, bevorzugt höchstens 200 pm, weiter bevorzugt höchstens 180 pm, bevorzugter 150 pm. Wenn das Verfahren mit mindestens zwei Molekülen I und II durchgeführt wird und die Länge des Abstands zwischen Atomen der verschiedenen Moleküle ausgewählt wird, so kann bevorzugt der Übergangszustand eine Synthesereaktion mit dem Verfahren berechnet werden. Bevorzugt weist Molekül I eine Größe von $\leq$ 100 Atomen auf und Molekül II eine Größe von $\leq$ 100 Atomen auf, bevorzugter weist Molekül I eine Größe von $\leq$ 80 Atomen auf und Molekül II eine Größe von $\leq$ 80 Atomen auf, noch bevorzugter weist Molekül I eine Größe von $\leq$ 60 Atomen auf und Molekül II eine Größe von $\leq$ 60 Atomen auf. Bevorzugt beträgt die Summe der Atome aus Molekül I und aus Molekül II $\leq$ 100 Atome.

Bevorzugt ist Molekül I ein Katalysator für die chemische Reaktion, insbesondere für eine Polymersynthese, und Molekül II ein Edukt der chemischen Reaktion. Dabei handelt es sich bei der Polymersynthese in dieser Ausführungsform bevorzugt um Polyurethansynthesen. Die chemische Reaktion in dieser Ausführungsform sind bevorzugt auch Synthesen von Monomeren für Polymerisationsreaktionen, industriell benötigte Grundchemikalien, Additiven, Tensiden und pharmakologischen Wirkstoffen. Insbesondere ist die chemische Reaktion eine Synthese für mit Katalysatoren erhaltene Grundchemikalien oder Edukte für chemische Synthesen. Unter Additiven werden allgemein Additive für Kunststoffe wie Weichmacher, Antioxidantien, Stellmittel verstanden, sowie Zusätze für Kraftstoffe, bei deren Synthese jeweils Katalysatoren verwendet werden.

In Schritt A3 wird die in Schritt A2 erhaltene ausgewählte Startgeometrie in kartesischen und/oder internen Koordinaten dargestellt. Interne Koordinaten beschreiben die räumliche Anordnung der Atome relativ zueinander unter Benutzung von Bindungslängen, Bindungswinkel und Torsionswinkel.

In **Schritt B** des erfindungsgemäßen Verfahrens wird eine optimierte Startgeometrie ermittelt. Dazu wird zunächst in Schritt B1 ein Funktionsraum festgelegt. Der Funktionsraum umfasst die Raumkoordinaten von ausgewählten Atomen, wobei die Raumkoordinaten im Vektorraum aller im Molekül beinhalteten Atomkoordinaten einen Unterraum aufspannen. Die ausgewählten Atome für den Funktionsraum sind ein Satz an Atomen, die an einer Bindungsdissoziation beteiligt sind oder bevorzugt an einer Synthesereaktion. Des Weiteren umfasst der Funktionsraum die mindestens eine Bindung aus Schritt A2. Die Bei der bevorzugten Ausführungsform mit mindestens zwei Molekülen I und II umfasst der Funktionsraum den Abstand zwischen mindestens einem Atom aus Molekül I und mindestens einem Atom aus Molekül II.

Im folgenden Schritt B2 wird die Startgeometrie einer Geometrieoptimierung mittels einer quantenchemischen Methode unterzogen mit der Randbedingung, dass die in Schritt A2 ausgewählte Länge der mindestens einen Bindung konstant gehalten wird, so dass eine optimierte Startgeometrie erhalten wird. Dabei umfasst die Geometrieoptimierung alle Atome des als Startgeometrie ausgewählten Moleküls. Bei der bevorzugten Ausführungsform mit mindestens zwei Molekülen I und II wird bei der Geometrieoptimierung der Abstand zwischen mindestens einem Atom aus Molekül I und mindestens einem Atom aus Molekül II konstant gehalten, so dass eine optimierte Startgeometrie erhalten wird.

Bei der Geometrieoptimierung mit Randbedingung wird die Gesamtenergie des Moleküls als Funktion der Kernkoordinaten der im Molekül enthaltenen Atome mit einem quantenchemischen Verfahren minimiert. Um ein lokales Energieminimum zu erhalten wird die Energie nach Gradienten minimiert (zb. Steepest Descent), wobei die Energie soweit minimiert wird, wie die Randbedingung dies zulässt.

In Schritt B3 wird die Gradient-Norm B3 für die zuvor erhaltene optimierte Startgeometrie ermittelt, wobei die Gradient-Norm durch die erste Ableitung einer Funktion E = f(x) mittels der quantenchemischen Methode erhalten wird, mit E=Gesamtenergie der optimierten Startgeometrie und x=Kernkoordinaten des Moleküls in der optimierten Startgeometrie. In Schritt B3 wird die gleiche quantenchemische Methode verwendet wie in Schritt B2. Die euklidische Norm dieses Vektors ist die Gradient-Norm. Mit anderen Worten wird der Gradient-Vektor der Energie berechnet. Der Gradient-Vektor spannt denselben vektoriellen Raum auf wie das Molekül. Jede Komponente des Vektors besteht aus der partiellen Ableitung der Energie in der $x_i$-Koordinate ( $\frac{\partial}{\partial x_i}E(x)$ ) und spannt sich in die Richtung des Einheitsvektors der $x_i$-Koordinate. Danach wird die (euklidische) Norm dieses Vektors ermittelt.

Die weitere Fortführung des Verfahrens von der Größe der erhaltenen Gradient-Norm B3 ab. Sofern die Gradient-Norm B3 $\nabla$ $0 \leq \nabla \leq 0,07$ $E_h$ $a_0^{-1}$ beträgt, dann kann die optimierten Startgeometrie als eine Vorstufe für den Übergangszustand der chemischen Reaktion eingeordnet werden und Schritt C des Verfahrens ausgelassen werden und das Verfahren mit Schritt D1 fortgeführt werden. Beträgt die Gradient-Norm B3 $\nabla > 0,07$ $E_h$ $a_0^{-1}$, dann wird Schritt C des Verfahrens durchgeführt und zunächst eine Vorstufe für den Übergangszustand der chemischen Reaktion ermittelt und zwar ausgehend von der optimierten Startgeometrie. Dabei steht $E_h$ für die Hartree Energie, wobei 1 $E_h$ = 4.3597 · 10$^{-18}$ J, und $a_0$ steht für den Bohr-Radius, wobei 1 $a_0$ = 5.29 · 10$^{-11}$ m.

In **Schritt C** wird die Vorstufe für den Übergangszustand der chemischen Reaktion ermittelt. Dazu wird die optimierte Startgeometrie unter Anwendung eines Monte-Carlo-Algorithmus variiert indem zufällig generierte Auslenkungen von Atomen in einem Ensemble, das aus einem Molekül oder bevorzugt einer Geometrie von mindestens zwei Molekülen I und II besteht, vorgenommen.

Monte-Carlo-Algorithmen oder -Verfahren sind Simulationsverfahren, die analytisch nicht oder nur schwer lösbare mathematische Probleme durch nummerische Näherungen lösen. Dabei wird ein wahrscheinlichster Verlauf eines Experiments durch eine Vielzahl an zufällig angeordneten Einzelexperimenten beschrieben.

In dem erfindungsgemäßen in Schritt C angewandten Monte-Carlo-Algorithmus werden zufällig generierte Auslenkungen von Atomen in einem Ensemble, das aus einem Molekül oder einer Geometrie von mindestens zwei Molekülen I und II besteht, vorgenommen und die Änderung einer Observablen beobachtet. Bei dieser Observablen handelt es sich um eine Gradient-Norm. Dabei variiert das erfindungsgemäße Verfahren nur einen Unterraum der deutlich niedriger dimensional ist als der gesamte Funktionsraum des Ensembles und betrachtet die Änderung der Observablen des gesamten Ensembles als Funktion des reduzierten Funktionsraums.

Dazu werden aus dem in Schritt B1 ausgewählten Funktionsraum, in Schritt C 1.1 mindestens ein Atom ausgewählt. Dies geschieht mit Hilfe einer Zufallszahl Z1. Die Richtung einer Auslenkung des durch Z1 gewählten Atoms, bzw. die Richtung des Vektors für die Auslenkung, wird in Schritt C1.2 durch eine weitere Zufallszahl Z2 ausgewählt. Die Richtung wird bevorzugt definiert, indem im bevorzugten Schritt C1.2a die Position eines anderen Atoms als dem in Schritt C1.1. ausgewählten aus dem reduzierten Funktionsraum ausgewählt wird und dann im bevorzugten Schritt C1.2b die Richtung der Auslenkung ausgewählt wird, wobei die Position des in Schritt C1.2a ausgewählten Atoms die Richtung des Vektors bestimmt. Die Amplitude der Auslenkung wird bevorzugt durch eine dritte Zufallszahl Z3 im bevorzugten Schritt C1.2c bestimmt.

In einer bevorzugten Ausführungsform des erfindungsgemäßen Verfahrens umfasst Schritt C1.2 die weiteren folgenden Schritte:

C1.2a Bestimmung eines weiteren Atoms aus dem in Schritt B1 ausgewählten Funktionsraum, das nicht mit dem in Schritt C1.1 ausgewählten Atom übereinstimmt,
C1.2b Auswahl der Richtung des Vektors, wobei die Position des in Schritt C1.2.a ausgewählten Atoms die Richtung des Vektors bestimmt,
C1.2.c zufällige Auswahl der Länge des Vektors, wobei der Wert für die Länge des Vektors positive oder negative Werte annehmen kann.

Dadurch dass die Position des in Schritt C1.2 ausgewählten Atoms die Richtung des Vektors bestimmt, wird der Funktionsraum möglichst stark beschränkt und diese Vorgehensweise führt auch dazu das vorrangig Bindungslängen variiert werden, die Längen aufweisen wie sie sehr wahrscheinlich tatsächlich während einer Reaktion erreicht werden.

In Schritt C1.3 wird das in Schritt C.1.1 ausgewählte Atom anhand des Vektors aus Schritt 1.2 aus seiner Position in der optimierten Startgeometrie ausgelenkt, so dass eine Vorstufe für den Übergangszustand der chemischen Reaktion erhalten wird.

Die erhaltene Vorstufe für den Übergangszustand wird in Schritt C2 einer Geometrieoptimierung mittels der quantenchemischen Methode unterzogen mit der Randbedingung, dass die mindestens eine Bindung aus Schritt A2 die Länge hat, die in Schritt C1.3 ermittelt wurde. In Schritt C2 wird die gleiche quantenchemische Methode verwendet wie in den Schritten B2 und B3. Dann wird in Schritt C3 die Gradient-Norm C3 für die Vorstufe für den Übergangszustand aus Schritt C2 ermittelt, wobei die Gradient-Norm durch die erste Ableitung der Funktion E = f(x) mittels der quantenchemischen Methode erhalten wird, mit E=Gesamtenergie der Vorstufe für den Übergangszustand und x=Kernkoordinaten des Moleküls in der Vorstufe für den Übergangszustand. In Schritt C3 wird die gleiche quantenchemische Methode verwendet wie in den Schritten B2, B3 und C2.

Die weitere Fortführung des Verfahrens von der Größe der erhaltenen Gradient-Norm C3 ab. Sofern die Gradient-Norm C3 $\nabla$ $0 \leq \nabla \leq 0{,}07$ $E_h$ $a_0^{-1}$ beträgt, dann kann Schritt D1 des Verfahrens mit der Vorstufe für den Übergangszustand durchgeführt werden. Dabei steht $E_h$ für die Hartree Energie, wobei 1 $E_h$ = 4.3597 · 10$^{-18}$ J, und $a_0$ steht für den Bohr-Radius, wobei 1 $a_0$ = 5.29 · 10$^{-11}$ m.

Beträgt die Gradient-Norm C3 $\nabla$ > 0,07 $E_h$ $a_0^{-1}$, dann werden in Schritt 4.2 die Schritte C1 bis C3 wiederholt bis eine Gradient-Norm C3 $\nabla$ $0 \leq \nabla \leq 0{,}07$ $E_h$ $a_0^{-1}$ erhalten wird. Durch die Wiederholung der Schritte C1 bis C3 wird ein iteratives Verfahren dargestellt, mit dem die Gradient-Norm minimiert werden soll. Daher soll als Ausgangspunkt für die Wiederholung des Verfahrens jeweils eine Molekülgeometrie gewählt werden, die bereits eine möglichst niedrige Gradient-Norm aufweist. Bei der Wiederholung der Schritte C1 bis C3 kann also in Schritt C1 nicht nur die optimierte Startgeometrie variiert werden, sondern auch eine Vorstufe für den Übergangszustand, sofern der Wert ihrer Gradient-Norm C3 kleiner ist als derjenige der optimierten Startgeometrie. Bevorzugt werden die bei der Durchführung des Verfahrens erhaltene optimierte Startgeometrie und erhaltene(n) Vorstufe(n) für den Übergangszustand sowie die Werte der jeweiligen Gradient-Normen C3 gespeichert.

In dem Fall, dass die Schritte C1 bis C3 ein Mal durchgeführt wurden, kann aus zwei Molekülgeometrien zur Durchführung der Wiederholung diejenige mit der niedrigsten Gradient-Norm ausgewählt werden, nämlich entweder die optimierte Startgeometrie oder die in ersten Durchgang erhaltene Vorstufe für den Übergangszustand. In dem Fall, dass die Schritte C1 bis C3 ein Mal durchgeführt wurden, wird in Schritt C1 die Geometrie der Vorstufe für den Übergangszustand variiert, wenn der Wert ihrer Gradient-Norm C3 niedriger ist als der Wert der Gradient-Norm B3 der optimierten Startgeometrie.

In dem Fall, dass die Schritte C1 bis C3 schon durchgeführt wurden, kann aus mehr als zwei Molekülgeometrien zur Durchführung der Wiederholung diejenige mit der niedrigsten Gradient-Norm ausgewählt werden, nämlich entweder die optimierte Startgeometrie oder die in bei den Wiederholungen erhaltenen (mehr als eine) Vorstufen für den Übergangszustand. In dem Fall dass die Schritte C1 bis C3 mehr als ein Mal durchgeführt wurden, wird in Schritt C1 die Geometrie der optimierten Startgeometrie oder derjenigen Vorstufe für den Übergangszustand aus den vorrangegangenen Wiederholungen variiert wird, welche im Vergleich zu allen bisher erhaltenen Gradient-Normen C3 und B3 den niedrigsten Wert für die Gradient-Norm C3 oder B3 aufweist.

Bevorzugt ist in Schritt C4.1 die Gradient-Norm C3 $\leq 0{,}05$ $E_h$ $a_0^{-1}$, bevorzugt C3 $\leq 0{,}04$ $E_h$ $a_0^{-1}$, bevorzugter $\leq 0{,}03$ $E_h$ $a_0^{-1}$, und Schritt C4.2 wird so lange durchgeführt, bis eine Gradient-Norm C3 $\leq 0{,}05$ $E_h$ $a_0^{-1}$, bevorzugt C3 $\leq 0{,}04$ $E_h$ $a_0^{-1}$, bevorzugter $\leq 0{,}03$ $E_h$ $a_0^{-1}$, erhalten wird.

Bevorzugt wird Schritt **C4.2** höchstens 50 Mal, bevorzugt höchstens 40 Mal, bevorzugter höchstens 30 Mal, wiederholt. Bevorzugt kann bei der Durchführung von Schritt **C4.2** bei der Wiederholung von Schritt **C1** ein anderes Atom ausgewählt werden als bei der vorrangegangenen Durchführung des Verfahrens.

Ein Vorteil des erfindungsgemäßen Verfahrens ist, dass das bei der Variation unter Anwendung eines Monte-Carlo-Algorithmus nur die Atomkoordinaten des ausgewählten Funktionsraums, also des Unterraums der durch die ausgewählten, an der betrachteten Bindungsdissoziation oder bevorzugt Synthesereaktion beteiligten Atome im Vektorraum aller im Molekül, oder im Molekülensemble, beinhalteten Atomkoordinaten aufgespannt wird. Das heißt, der erfindungsgemäße Monte-Carlo-Algorithmus operiert nur in diesem Unterraum, da seine Funktionen nur hierin definiert sind. Damit ist der Unterraum der an der Dissoziation, oder bevorzugten Synthesereaktion, beteiligten Koordinaten der Funktionsraum des Monte-Carlo-Algorithmus. Die Berechnung der Energien und der Gradient-Vektoren (bzw. deren Norm) hängen zwar von den gesamten Koordinaten im Molekül ab, jedoch betrachtet der erfindungsgemäße Monte-Carlo-Algorithmus diese nur in Abhängigkeit der Koordinaten des Unterraumes. Damit bei dieser Betrachtung der Funktionswert, die zu minimierende Gradient-Norm, eindeutig bleibt, wird vor jeder Betrachtung des Funktionswerts die gesamte Molekülstruktur durch eine Geometrieoptimierung mit Randbedingung(en) relaxiert. Die Randbedingung(en) sind dabei die durch den Monte-Carlo-Algorithmus erzeugten Bindungsabstände.

**In Schritt D1** wird die Vorstufe aus Schritt C4.1 oder die Vorstufe aus Schritt B4.1 mittels der quantenchemischen Methode und eines Pseudo-Newton-Raphson-Algorithmus einer relaxiert, so dass der Übergangszustand erhalten wird. In Schritt D1 wird die gleiche quantenchemische Methode verwendet wie in den Schritten B2, B3, C2 und C3.

Dazu wird bevorzugt zur Überführung der jeweiligen Molekülgeometrie in einen Stationären Punkt (G=0) wird eine Geometrieoptimierung vorgenommen, bei der die Gesamtenergie als Funktion der Kernkoordinaten der im Molekül enthaltenen Atome minimiert wird. Um ein lokales Energieminimum zu erhalten wird bevorzugt die Energie nach Gradienten minimiert (zb. Steepest Descent). Um Sattelpunkte erster Ordnung (chemisch als Übergangszustände interpretierbar) zu erhalten wird ein Newton-Raphson Algorithmus verwendet.

In Schritt D2 wird gegebenenfalls Ermittlung eines Gleichgewichtszustands durch Auslenkung des Übergangszustands, so dass ein ausgelenkter Übergangszustand erhalten wird und Relaxation des ausgelenkten Übergangszustands mittels der quantenchemischen Methode, so dass ein Gleichgewichtszustand erhalten wird. In Schritt D2 wird die gleiche quantenchemische Methode verwendet wie in den Schritten B2, B3, C2, C3 und D1.

Der oben beschriebenen Vorgehensweise in den Schritten C und D liegen folgende grundsätzliche Überlegungen zugrunde: Der Übergangszustand ist charakterisiert durch eine Sattelpunktstruktur des Reaktionspfades mit einem Gradienten der Größe Null und einer negativen Krümmung entlang eines geometrischen Freiheitsgrades. Sollen die nächst liegenden lokalen Energieminima zur Sattelpunktstruktur erhalten werden (chemisch interpretierbar als Edukte, Produkte oder Intermediate), kann eine geometrische Störung der Sattelpunkt-Struktur d.h. eine Auslenkung der Atome des Moleküls, vorgenommen werden, so dass eine Struktur mit nicht verschwindendem Gradienten erhalten wird. Wird diese Molekülstruktur einer Geometrieoptimierung nach Gradienten unterzogen, können damit lokale Minima erhalten werden, die an den Sattelpunkt angrenzen.

In den Schritten **B2, B3, C2, C3, D1** und **D2** wird jeweils dieselbe quantenchemische Methode verwendet. Bevorzugt ist die quantenchemische Methode aus Schritten **B2, B3, C2, C3, D1** und **D2** eine semiempirische Methode, Dichte-Funktional-theoretische Methode oder eine Annäherung der Schrödinger-Gleichung, insbesondere sind Dichte-Funktional-theoretische Methoden bevorzugt, wie beispielsweise das TPSS-Dichtefunktional mit einem def2-SVP-Basissatz, so wie standardmäßig im Turbomole-Programmpaket implementiert. In einer bevorzugten Ausführungsform werden die quantenmechanischen Berechnungen mit dem Programmpaket TURBOMOLE durchgeführt. Bevorzugt wird zur Berechnung ein Computer mit 16 Core-Prozessoren mit einer Taktfrequenz von 3.20GHz und 25MB Zwischenspeicher mit einem RAM Speicher von 128 GB DDR4 2400 rg ECC verwendet.

Bevorzugt ist die chemische Reaktion eine Synthese ausgewählt aus der Gruppe bestehend aus Polymersynthesen, insbesondere Polyurethansynthesen, Synthesen von Monomeren für Polymerisationsreaktionen, industriell benötigte Grundchemikalien, Additiven, Tensiden und pharmakologischen Wirkstoffen. Insbesondere ist die chemische Reaktion eine Synthese für mit Katalysatoren erhaltene Grundchemikalien oder Edukte für chemische Synthesen. Unter Additiven werden allgemein Additive für Kunststoffe wie Weichmacher, Antioxidantien, Stellmittel verstanden, sowie Zusätze für Kraftstoffe, bei deren Synthese jeweils Katalysatoren verwendet werden.

Wenn bei der bevorzugten Ausführungsform des Verfahrens mindestens zwei Moleküle I und II bereitgestellt werden, können bevorzugt die unter den Buchstaben **A, B,** und **C** des Verfahrens genannten Schritte wiederholt werden, wobei bei jeder Wiederholung im Vergleich zur vorrangegangenen Durchführungen des Verfahrens

Molekül I verändert wird oder ein anderes Molekül als Molekül I bereitgestellt wird, und Molekül II nicht verändert wird und auch kein anderes Molekül als Molekül II bereitgestellt wird,
und der zusätzlichen Schritt D0 durchgeführt werden:
**D0** Vergleich der bei den Wiederholungen erhaltenen Gradienten-Norm C3 für die verschiedenen Vorstufen für den Übergangszustand und Auswahl der Vorstufe für den Übergangszustand mit der niedrigsten Gradienten-Norm C3 und Durchführung von Schritt D1 und/oder D2 mit der ausgewählten Vorstufe für den Übergangszustand.

Bei dieser zuvor beschriebenen bevorzugten Ausführungsform des Verfahrens werden zunächst für unterschiedliche Kombinationen von Molekülen die Gradient-Norm C3 berechnet und die Ergebnisse gespeichert. Dann können in einem bevorzugten Schritt D0 die erhaltenen Werte für die Gradient-Norm C3 verglichen werden und die Kombination von Molekülen mit der niedrigsten Gradienten-Norm C3 ausgewählt werden. Diese bevorzugte Ausführungsform des Verfahrens ermöglicht also den direkten Vergleich von unterschiedlichen Kombinationen von Molekülen.

In einer weiteren alternativ-bevorzugten Ausführungsform betrifft die Erfindung ein Verfahren wobei Information über den gemäß Schritt D.1 ermittelten Übergangszustand und/oder der gemäß Schritt D.2 ermittelten Gleichgewichtszustand einem Benutzer mitgeteilt wird.

In einer weiteren alternativ-bevorzugten Ausführungsform betrifft die Erfindung ein Verfahren wobei Information über den gemäß Schritt D.1 ermittelten Übergangszustand und/oder der gemäß Schritt D.2 ermittelten Gleichgewichtszustand durch einen Benutzer empfangen wird.

In einer weiteren alternativ-bevorzugten Ausführungsform betrifft die Erfindung ein Verfahren wobei nach Schritt D.1 und/oder nach Schritt D.2 das Molekül I synthetisiert wird.

In einer weiteren alternativ-bevorzugten Ausführungsform betrifft die Erfindung ein Verfahren wobei nach Schritt D.1 und/oder nach Schritt D.2 unter eine chemische Reaktion mit dem Molekül I als Katalysator durchgeführt wird.

In einer weiteren alternativ-bevorzugten Ausführungsform betrifft die Erfindung ein Verfahren wobei nach Schritt D.1 und/oder nach Schritt D.2 unter eine chemische Reaktion mit dem Molekül II als Reaktionspartner durchgeführt wird.

Ein weiterer Gegenstand der Erfindung ist ein System zur Datenverarbeitung umfassend Mittel zur Ausführung eines erfindungsgemäßen Verfahrens.

Darüber hinaus ist ein Gegenstand der Erfindung ein Computerprogramm, umfassend Befehle, die bei der Ausführung des Programms durch einen Computer diesen veranlassen, die folgenden Schritte eines Verfahrens durchzuführen:

**A Erzeugen einer Startgeometrie**

A1 Bereitstellung der dreidimensionalen Darstellung mindestens eines Moleküls im energetischen Grundzustand,
A2 Auswahl von mindestens einer Bindung des mindestens einen Moleküls und Auswahl einer Länge der Bindung, wobei die ausgewählte Länge nicht der Länge der Bindung im energetischen Grundzustand des Moleküls entspricht, so dass eine Startgeometrie für die chemische Reaktion erhalten wird,
A3 Dreidimensionale Darstellung der Startgeometrie in kartesischen und/oder internen Koordinaten,

**B Ermittlung einer optimierten Startgeometrie**

B1 Festlegung eines Funktionsraumes welcher die mindestens eine Bindung aus Schritt A2 und die durch diese Bindung verbundenen Atome umfasst,
B2 Geometrieoptimierung der Startgeometrie unter Zugrundelegung des in Schritt B1 ausgewählten Funktionsraums mittels einer quantenchemischen Methode und mit der Randbedingung, dass die in Schritt A2 ausgewählte Länge der mindestens einen Bindung konstant gehalten wird, so dass eine optimierte Startgeometrie erhalten wird,

B3 Ermittlung der Gradient-Norm B3 für die optimierte Startgeometrie, wobei die Gradient-Norm durch die erste Ableitung einer Funktion E = f(x) mittels der quantenchemischen Methode erhalten wird, mit E=Gesamtenergie der optimierten Startgeometrie und x=Kernkoordinaten des Moleküls in der optimierten Startgeometrie,
B4.1 wenn die Gradient-Norm B3 $\nabla$ $0 \leq \nabla \leq 0{,}07$ $E_h$ $a_0^{-1}$ beträgt, dann Einordnung der optimierten Startgeometrie als eine Vorstufe für den Übergangszustand der chemischen Reaktion und Fortführung des Verfahrens mit Schritt D1, oder
B4.2 wenn die Gradient-Norm B3 $\nabla$ > $0{,}07$ $E_h$ $a_0^{-1}$ beträgt, dann Ermittlung der Vorstufe für den Übergangszustand der chemischen Reaktion ausgehend von der optimierten Startgeometrie durch ein Verfahren umfassend die folgenden Schritte:

**C Ermittlung der Vorstufe für den Übergangszustand der chemischen Reaktion**

C1 Variation der optimierten Startgeometrie unter Anwendung eines Monte-Carlo-Algorithmus wobei
C1.1 wenigstens ein Atom aus dem in Schritt B1 ausgewählten Funktionsraum zufällig ausgewählt wird,
C1.2 ein Vektor für eine Auslenkung des in Schritt C1.1 gewählten Atoms zufällig ausgewählt wird,
C1.3 das in Schritt C1.1 ausgewählte Atom anhand des Vektors aus Schritt C1.2 aus seiner Position in der optimierten Startgeometrie ausgelenkt wird, so dass eine Vorstufe für den Übergangszustand der chemischen Reaktion erhalten wird,
C2 Geometrieoptimierung der Vorstufe für den Übergangszustand mittels der quantenchemischen Methode und mit der Randbedingung, dass die mindestens eine Bindung aus Schritt A2 die Länge hat, die in der Schritt C1.3 ermittelt wurde,
C3 Ermittlung der Gradient-Norm C3 für die Vorstufe für den Übergangszustand aus Schritt C2, wobei die Gradient-Norm durch die erste Ableitung der Funktion E = f(x) mittels der quantenchemischen Methode erhalten wird, mit E=Gesamtenergie der Vorstufe für den Übergangszustand und x=Kernkoordinaten des Moleküls in der Vorstufe für den Übergangszustand,
C4.1 wenn die Gradient-Norm C3 $\nabla$ $0 \leq \nabla \leq 0{,}07$ $E_h$ $a_0^{-1}$ beträgt, dann Fortführung des Verfahrens mit Schritt D1, oder
C4.2 wenn die Gradient-Norm C3 $\nabla$ > $0{,}07$ $E_h$ $a_0^{-1}$ beträgt, dann Wiederholung der Schritte C1 bis C3 bis eine Gradient-Norm C3 $0 \leq \nabla \leq 0{,}07$ $E_h$ $a_0^{-1}$ erhalten wird, wobei

(a) für den Fall, dass die Schritte C1 bis C3 ein Mal durchgeführt wurden, in Schritt C1 die Geometrie der Vorstufe für den Übergangszustand variiert wird, wenn der Wert ihrer Gradient-Norm C3 niedriger ist als der Wert der Gradient-Norm B3 der optimierten Startgeometrie, oder

(b)für den Fall dass die Schritte C1 bis C3 mehr als ein Mal durchgeführt wurden, in Schritt C1 die Geometrie der optimierten Startgeometrie oder derjenigen Vorstufe für den Übergangszustand aus den vorrangegangen Wiederholungen variiert wird, welche im Vergleich zu allen bisher erhaltenen Gradient-Normen C3 und B3 den niedrigsten Wert für die Gradient-Norm C3 oder B3 aufweist,

## D Ermittlung des Übergangszustandes

D1 Relaxation der Vorstufe aus Schritt C4.1 oder der Vorstufe aus Schritt B4.1 mittels der quantenchemischen Methode und eines Pseudo-Newton-Raphson-Algorithmus, so dass der Übergangszustand erhalten wird,
D2 gegebenenfalls Ermittlung eines Gleichgewichtszustands durch Auslenkung des Übergangszustands, so dass ein ausgelenkter Übergangszustand erhalten wird und Relaxation des ausgelenkten Übergangszustands mittels der quantenchemischen Methode, so dass ein Gleichgewichtszustand erhalten wird.

Bevorzugt umfasst das Computerprogramm Befehle, die bei der Ausführung des Programms durch einen Computer diesen veranlassen, die Schritte B bis D des Verfahrens durchzuführen.
Außerdem ist ein weiterer Gegenstand der Erfindung ein computerlesbares Speichermedium, umfassend Befehle, die bei der Ausführung durch einen Computer diesen veranlassen, die folgenden Schritte eines Verfahrens durchzuführen:

## A Erzeugen einer Startgeometrie

A1 Bereitstellung der dreidimensionalen Darstellung mindestens eines Moleküls im energetischen Grundzustand,
A2 Auswahl von mindestens einer Bindung des mindestens einen Moleküls und Auswahl einer Länge der Bindung, wobei die ausgewählte Länge nicht der Länge der Bindung im energetischen Grundzustand des Moleküls entspricht, so dass eine Startgeometrie für die chemische Reaktion erhalten wird,
A3 Dreidimensionale Darstellung der Startgeometrie in kartesischen und/oder internen Koordinaten,

## B Ermittlung einer optimierten Startgeometrie

B1 Festlegung eines Funktionsraumes welcher die mindestens eine Bindung aus Schritt A2 und die durch diese Bindung verbundenen Atome umfasst,
B2 Geometrieoptimierung der Startgeometrie unter Zugrundelegung des in Schritt B1 ausgewählten Funktionsraums mittels einer quantenchemischen Methode und mit der Randbedingung, dass die in Schritt A2 ausgewählte Länge der mindestens einen Bindung konstant gehalten wird, so dass eine optimierte Startgeometrie erhalten wird,

B3 Ermittlung der Gradient-Norm B3 für die optimierte Startgeometrie, wobei die Gradient-Norm durch die erste Ableitung einer Funktion E = f(x) mittels der quantenchemischen Methode erhalten wird, mit E=Gesamtenergie der optimierten Startgeometrie und x=Kernkoordinaten des Moleküls in der optimierten Startgeometrie,
B4.1 wenn die Gradient-Norm B3 $\nabla$ $0 \leq \nabla \leq 0{,}07$ $E_h\,a_0^{-1}$ beträgt, dann Einordnung der optimierten Startgeometrie als eine Vorstufe für den Übergangszustand der chemischen Reaktion und Fortführung des Verfahrens mit Schritt D1, oder
B4.2 wenn die Gradient-Norm B3 $\nabla > 0{,}07$ $E_h\,a_0^{-1}$ beträgt, dann Ermittlung der Vorstufe für den Übergangszustand der chemischen Reaktion ausgehend von der optimierten Startgeometrie durch ein Verfahren umfassend die folgenden Schritte:

## C Ermittlung der Vorstufe für den Übergangszustand der chemischen Reaktion

C1 Variation der optimierten Startgeometrie unter Anwendung eines Monte-Carlo-Algorithmus wobei
C1.1 wenigstens ein Atom aus dem in Schritt B1 ausgewählten Funktionsraum zufällig ausgewählt wird,
C1.2 ein Vektor für eine Auslenkung des in Schritt C1.1 gewählten Atoms zufällig ausgewählt wird,

C1.3 das in Schritt C1.1 ausgewählte Atom anhand des Vektors aus Schritt C1.2 aus seiner Position in der optimierten Startgeometrie ausgelenkt wird, so dass eine Vorstufe für den Übergangszustand der chemischen Reaktion erhalten wird,

C2 Geometrieoptimierung der Vorstufe für den Übergangszustand mittels der quantenchemischen Methode und mit der Randbedingung, dass die mindestens eine Bindung aus Schritt A2 die Länge hat, die in der Schritt C1.3 ermittelt wurde,

C3 Ermittlung der Gradient-Norm C3 für die Vorstufe für den Übergangszustand aus Schritt C2, wobei die Gradient-Norm durch die erste Ableitung der Funktion E = f(x) mittels der quantenchemischen Methode erhalten wird, mit E=Gesamtenergie der Vorstufe für den Übergangszustand und x=Kernkoordinaten des Moleküls in der Vorstufe für den Übergangszustand,

C4.1 wenn die Gradient-Norm C3 $\nabla$ $0 \leq \nabla \leq 0,07$ $E_h\,a_0^{-1}$ beträgt, dann Fortführung des Verfahrens mit Schritt D1, oder C4.2 wenn die Gradient-Norm C3 $\nabla > 0,07$ $E_h\,a_0^{-1}$ beträgt, dann Wiederholung der Schritte C1 bis C3 bis eine Gradient-Norm C3 $0 \leq \nabla \leq 0,07$ $E_h\,a_0^{-1}$ erhalten wird, wobei

(a) für den Fall, dass die Schritte C1 bis C3 ein Mal durchgeführt wurden, in Schritt C1 die Geometrie der Vorstufe für den Übergangszustand variiert wird, wenn der Wert ihrer Gradient-Norm C3 niedriger ist als der Wert der Gradient-Norm B3 der optimierten Startgeometrie, oder

(b) für den Fall dass die Schritte C1 bis C3 mehr als ein Mal durchgeführt wurden, in Schritt C1 die Geometrie der optimierten Startgeometrie oder derjenigen Vorstufe für den Übergangszustand aus den vorrangegangen Wiederholungen variiert wird, welche im Vergleich zu allen bisher erhaltenen Gradient-Normen C3 und B3 den niedrigsten Wert für die Gradient-Norm C3 oder B3 aufweist,

**D Ermittlung des Übergangszustandes**

D1 Relaxation der Vorstufe aus Schritt C4.1 oder der Vorstufe aus Schritt B4.1 mittels der quantenchemischen Methode und eines Pseudo-Newton-Raphson-Algorithmus, so dass der Übergangszustand erhalten wird,

D2 gegebenenfalls Ermittlung eines Gleichgewichtszustands durch Auslenkung des Übergangszustands, so dass ein ausgelenkter Übergangszustand erhalten wird und Relaxation des ausgelenkten Übergangszustands mittels der quantenchemischen Methode, so dass ein Gleichgewichtszustand erhalten wird.

Bevorzugt umfasst das computerlesbare Speichermedium Befehle, die bei der Ausführung des Programms durch einen Computer diesen veranlassen, die Schritte B bis D des Verfahrens durchzuführen. Hierbei kann das computerlesbare Speichermedium beispielsweise einen oder mehrere physikalisch existente Festplatte(n) geeignet zur Speicherung von Programmen zur Ausführung von Befehlen.

Des Weiteren bezieht sich die Erfindung auf die Verwendung des erfindungsgemäßen Computerprogramms oder des erfindungsgemäßen computerlesbaren Speichermediums zur Bewertung von Übergangszuständen einer chemischen Reaktion, insbesondere einer Polymersynthese.

Die Erfindung betrifft insbesondere die folgenden Ausführungsformen:

Nach einer ersten Ausführungsform betrifft die Erfindung ein computerimplementiertes Verfahren zur Berechnung von Übergangszuständen einer chemischen Reaktion umfassend die Schritte

**A Erzeugen einer Startgeometrie**

A1 Bereitstellung der dreidimensionalen Darstellung mindestens eines Moleküls im energetischen Grundzustand,

A2 Auswahl von mindestens einer Bindung des mindestens einen Moleküls und Auswahl einer Länge der Bindung, wobei die ausgewählte Länge nicht der Länge der Bindung im energetischen Grundzustand des Moleküls entspricht, so dass eine Startgeometrie für die chemische Reaktion erhalten wird,

A3 Dreidimensionale Darstellung der Startgeometrie in kartesischen und/oder internen Koordinaten,

**B Ermittlung einer optimierten Startgeometrie**

B1 Festlegung eines Funktionsraumes welcher die mindestens eine Bindung aus Schritt A2 und die durch diese Bindung verbundenen Atome umfasst,

B2 Geometrieoptimierung der Startgeometrie unter Zugrundelegung des in Schritt B1 ausgewählten Funktionsraums mittels einer quantenchemischen Methode und mit der Randbedingung, dass die in Schritt A2 ausgewählte Länge der mindestens einen Bindung konstant gehalten wird, so dass eine optimierte Startgeometrie erhalten wird,

B3 Ermittlung der Gradient-Norm B3 für die optimierte Startgeometrie, wobei die Gradient-Norm durch die erste Ableitung einer Funktion E = f(x) mittels der quantenchemischen Methode erhalten wird, mit E=Gesamtenergie der optimierten Startgeometrie und x=Kernkoordinaten des Moleküls in der optimierten Startgeometrie,

B4.1 wenn die Gradient-Norm B3 $\nabla$ $0 \leq \nabla \leq 0{,}07$ $E_h\,a_0^{-1}$ beträgt, dann Einordnung der optimierten Startgeometrie als eine Vorstufe für den Übergangszustand der chemischen Reaktion und Fortführung des Verfahrens mit Schritt D1, oder

B4.2 wenn die Gradient-Norm B3 $\nabla > 0{,}07$ $E_h\,a_0^{-1}$ beträgt, dann Ermittlung der Vorstufe für den Übergangszustand der chemischen Reaktion ausgehend von der optimierten Startgeometrie durch ein Verfahren umfassend die folgenden Schritte:

**C Ermittlung der Vorstufe für den Übergangszustand der chemischen Reaktion**

C1 Variation der optimierten Startgeometrie unter Anwendung eines Monte-Carlo-Algorithmus wobei

C1.1 wenigstens ein Atom aus dem in Schritt B1 ausgewählten Funktionsraum zufällig ausgewählt wird,

C1.2 ein Vektor für eine Auslenkung des in Schritt C1.1 gewählten Atoms zufällig ausgewählt wird,

C1.3 das in Schritt C1.1 ausgewählte Atom anhand des Vektors aus Schritt C1.2 aus seiner Position in der optimierten Startgeometrie ausgelenkt wird, so dass eine Vorstufe für den Übergangszustand der chemischen Reaktion erhalten wird,

C2 Geometrieoptimierung der Vorstufe für den Übergangszustand mittels der quantenchemischen Methode und mit der Randbedingung, dass die mindestens eine Bindung aus Schritt A2 die Länge hat, die in der Schritt C1.3 ermittelt wurde,

C3 Ermittlung der Gradient-Norm C3 für die Vorstufe für den Übergangszustand aus Schritt C2, wobei die Gradient-Norm durch die erste Ableitung der Funktion E = f(x) mittels der quantenchemischen Methode erhalten wird, mit E=Gesamtenergie der Vorstufe für den Übergangszustand und x=Kernkoordinaten des Moleküls in der Vorstufe für den Übergangszustand,

C4.1 wenn die Gradient-Norm C3 $\nabla$ $0 \leq \nabla \leq 0{,}07$ $E_h\,a_0^{-1}$ beträgt, dann Fortführung des Verfahrens mit Schritt D1, oder

C4.2 wenn die Gradient-Norm C3 $\nabla > 0{,}07$ $E_h\,a_0^{-1}$ beträgt, dann Wiederholung der Schritte C1 bis C3 bis eine Gradient-Norm C3 $0 \leq \nabla \leq 0{,}07$ $E_h\,a_0^{-1}$ erhalten wird, wobei

(a) für den Fall, dass die Schritte C1 bis C3 ein Mal durchgeführt wurden, in Schritt C1 die Geometrie der Vorstufe für den Übergangszustand variiert wird, wenn der Wert ihrer Gradient-Norm C3 niedriger ist als der Wert der Gradient-Norm B3 der optimierten Startgeometrie, oder

(b) für den Fall dass die Schritte C1 bis C3 mehr als ein Mal durchgeführt wurden, in Schritt C1 die Geometrie der optimierten Startgeometrie oder derjenigen Vorstufe für den Übergangszustand aus den vorrangegangen Wiederholungen variiert wird, welche im Vergleich zu allen bisher erhaltenen Gradient-Normen C3 und B3 den niedrigsten Wert für die Gradient-Norm C3 oder B3 aufweist,

**D Ermittlung des Übergangszustandes**

D1 Relaxation der Vorstufe aus Schritt C4.1 oder der Vorstufe aus Schritt B4.1 mittels der quantenchemischen Methode und eines Pseudo-Newton-Raphson-Algorithmus, so dass der Übergangszustand erhalten wird,

D2 gegebenenfalls Ermittlung eines Gleichgewichtszustands durch Auslenkung des Übergangszustands, so dass ein ausgelenkter Übergangszustand erhalten wird und Relaxation des ausgelenkten Übergangszustands mittels der quantenchemischen Methode, so dass ein Gleichgewichtszustand erhalten wird.

In einer zweiten Ausführungsform betrifft die Erfindung ein Verfahren nach Ausführungsform 1, dadurch gekennzeichnet, dass das mindestens eine Molekül aus Schritt A1 eine Größe von höchstens 100 Atomen aufweist und/oder dass die Länge der in Schritt **A2** ausgewählten Bindung höchstens 230 pm beträgt.

In einer dritten Ausführungsform betrifft die Erfindung ein Verfahren nach Ausführungsform 1 oder 2, dadurch gekennzeichnet, dass Schritt **C1.2** die weiteren folgenden Schritte umfasst:

C1.2a Bestimmung eines weiteren Atoms aus dem in Schritt B1 ausgewählten Funktionsraum, das nicht mit dem in Schritt C1.1 ausgewählten Atom übereinstimmt,
C1.2b Auswahl der Richtung des Vektors, wobei die Position des in Schritt C1.2.a ausgewählten Atoms die Richtung des Vektors bestimmt,
C1.2.c zufällige Auswahl der Länge des Vektors, wobei der Wert für die Länge des Vektors positive oder negative Werte annehmen kann.

In einer vierten Ausführungsform betrifft die Erfindung ein Verfahren nach einer der vorstehenden Ausführungsformen, dadurch gekennzeichnet, dass in Schritt **C4.1** die Gradient-Norm $C3 \leq 0,05\ E_h\ a_0^{-1}$, bevorzugt $C3 \leq 0,04\ E_h\ a_0^{-1}$, bevorzugter $\leq 0,03\ E_h\ a_0^{-1}$, ist und Schritt **C4.2** so lange durchgeführt wird, bis eine Gradient-Norm $C3 \leq 0,05\ E_h\ a_0^{-1}$, bevorzugt $C3 \leq 0,04\ E_h\ a_0^{-1}$, bevorzugter $\leq 0,03\ E_h\ a_0^{-1}$, erhalten wird.

In einer fünften Ausführungsform betrifft die Erfindung ein Verfahren nach einer der vorstehenden Ausführungsformen, dadurch gekennzeichnet, dass Schritt **C4.2** höchstens 50 Mal, bevorzugt höchstens 30 Mal, wiederholt wird.

In einer sechsten Ausführungsform betrifft die Erfindung ein Verfahren nach einer der vorstehenden Ausführungsformen dadurch gekennzeichnet, dass bei der Durchführung von Schritt **C4.2** bei der Wiederholung von Schritt **C1** ein anderes Atom ausgewählt werden kann als bei der vorrangegangenen Durchführung des Verfahrens.

In einer siebten Ausführungsform betrifft die Erfindung ein Verfahren nach einer der vorstehenden Ausführungsformen, dadurch gekennzeichnet, dass die quantenchemische Methode aus Schritten **B2, B3, C2, C3, D1** und **D2** eine semiempirische Methode, Dichte-Funktional-theoretische Methode oder eine Annäherung der Schrödinger-Gleichung ist, insbesondere ist die quantenchemische Methode aus Schritten **B2, B3, C2, C3, D1** und **D2** eine Dichte-Funktional-theoretische Methode.

In einer achten Ausführungsform betrifft die Erfindung ein Verfahren nach einer der vorstehenden Ausführungsformen, dadurch gekennzeichnet, dass die chemische Reaktion eine Synthese ist ausgewählt aus der Gruppe bestehend aus Polymersynthesen, insbesondere Polyurethansynthesen, Synthesen von Monomeren für Polymerisationsreaktionen, industriell benötigte Grundchemikalien, Additiven, Tensiden und pharmakologischen Wirkstoffen.

In einer neunten Ausführungsform betrifft die Erfindung ein Verfahren nach einer der vorstehenden Ausführungsformen, dadurch gekennzeichnet, dass in Schritt **A1** mindestens zwei Moleküle I und II bereitgestellt werden und in Schritt **A2** alternativ oder zusätzlich zu der mindestens einen Bindung auch mindestens ein Abstand zwischen mindestens einem Atom aus Molekül I und mindestens einem Atom aus Molekül II sowie die Länge des mindestens einen Abstands ausgewählt werden können, wobei die Länge des Abstands insbesondere höchstens 230 pm beträgt.

In einer zehnten Ausführungsform betrifft die Erfindung ein Verfahren nach Ausführungsform 9, dadurch gekennzeichnet, dass die unter den Buchstaben **A, B,** und **C** des Verfahrens genannten Schritte wiederholt werden, wobei bei jeder Wiederholung im Vergleich zur vorrangegangenen Durchführungen des Verfahrens

Molekül I verändert wird oder ein anderes Molekül als Molekül I bereitgestellt wird, und Molekül II nicht verändert wird und auch kein anderes Molekül als Molekül II bereitgestellt wird,
und umfassend einen zusätzlichen Schritt
**D0** Vergleich der bei den Wiederholungen erhaltenen Gradienten-Norm C3 für die verschiedenen Vorstufen für den Übergangszustand und Auswahl der Vorstufe für den Übergangszustand mit der niedrigsten Gradient-Norm C3 und Durchführung von Schritt D1 und/oder D2 mit der ausgewählten Vorstufe für den Übergangszustand.

In einer elften Ausführungsform betrifft die Erfindung ein Verfahren nach einer der Ausführungsformen 9 oder 10, dadurch gekennzeichnet, dass Molekül I ein Katalysator für die chemische Reaktion ist, insbesondere für eine Polymersynthese, und Molekül II ein Edukt der chemischen Reaktion.

In einer zwölften Ausführungsform betrifft die Erfindung ein Verfahren nach einer der Ausführungsformen 9 bis 11, dadurch gekennzeichnet, dass die Länge des in Schritt **A2** ausgewählten Abstands zwischen mindestens einem Atom aus Molekül I und/oder mindestens einem Atom aus Molekül II insbesondere höchstens 230 pm beträgt.

In einer dreizehnten Ausführungsform betrifft die Erfindung ein Verfahren nach einer der Ausführungsformen 9 bis 12, dadurch gekennzeichnet, dass Molekül I eine Größe von $\leq 100$ Atomen aufweist und Molekül II eine Größe von $\leq 100$ Atomen aufweist, bevorzugt soll die Summe der Atome aus Molekül I und aus Molekül II $\leq 100$ Atome betragen.

In einer vierzehnten Ausführungsform betrifft die Erfindung ein System zur Datenverarbeitung umfassend Mittel zur Ausführung eines Verfahrens umfassend die Schritte:

### A Erzeugen einer Startgeometrie

A1 Bereitstellung der dreidimensionalen Darstellung mindestens eines Moleküls im energetischen Grundzustand,
A2 Auswahl von mindestens einer Bindung des mindestens einen Moleküls und Auswahl einer Länge der Bindung, wobei die ausgewählte Länge nicht der Länge der Bindung im energetischen Grundzustand des Moleküls entspricht, so dass eine Startgeometrie für die chemische Reaktion erhalten wird,
A3 Dreidimensionale Darstellung der Startgeometrie in kartesischen und/oder internen Koordinaten,

### B Ermittlung einer optimierten Startgeometrie

B1 Festlegung eines Funktionsraumes welcher die mindestens eine Bindung aus Schritt A2 und die durch diese Bindung verbundenen Atome umfasst,
B2 Geometrieoptimierung der Startgeometrie unter Zugrundelegung des in Schritt B1 ausgewählten Funktionsraums mittels einer quantenchemischen Methode und mit der Randbedingung, dass die in Schritt A2 ausgewählte Länge der mindestens einen Bindung konstant gehalten wird, so dass eine optimierte Startgeometrie erhalten wird,
B3 Ermittlung der Gradient-Norm B3 für die optimierte Startgeometrie, wobei die Gradient-Norm durch die erste Ableitung einer Funktion E = f(x) mittels der quantenchemischen Methode erhalten wird, mit E=Gesamtenergie der optimierten Startgeometrie und x=Kernkoordinaten des Moleküls in der optimierten Startgeometrie,
B4.1 wenn die Gradient-Norm B3 $\nabla$ $0 \leq \nabla \leq 0{,}07$ $E_h$ $a_0^{-1}$ beträgt, dann Einordnung der optimierten Startgeometrie als eine Vorstufe für den Übergangszustand der chemischen Reaktion und Fortführung des Verfahrens mit Schritt D1, oder
B4.2 wenn die Gradient-Norm B3 $\nabla > 0{,}07$ $E_h$ $a_0^{-1}$ beträgt, dann Ermittlung der Vorstufe für den Übergangszustand der chemischen Reaktion ausgehend von der optimierten Startgeometrie durch ein Verfahren umfassend die folgenden Schritte:

### C Ermittlung der Vorstufe für den Übergangszustand der chemischen Reaktion

C1 Variation der optimierten Startgeometrie unter Anwendung eines Monte-Carlo-Algorithmus wobei
C1.1 wenigstens ein Atom aus dem in Schritt B1 ausgewählten Funktionsraum zufällig ausgewählt wird,
C1.2 ein Vektor für eine Auslenkung des in Schritt C1.1 gewählten Atoms zufällig ausgewählt wird,
C1.3 das in Schritt C1.1 ausgewählte Atom anhand des Vektors aus Schritt C1.2 aus seiner Position in der optimierten Startgeometrie ausgelenkt wird, so dass eine Vorstufe für den Übergangszustand der chemischen Reaktion erhalten wird,
C2 Geometrieoptimierung der Vorstufe für den Übergangszustand mittels der quantenchemischen Methode und mit der Randbedingung, dass die mindestens eine Bindung aus Schritt A2 die Länge hat, die in der Schritt C1.3 ermittelt wurde,
C3 Ermittlung der Gradient-Norm C3 für die Vorstufe für den Übergangszustand aus Schritt C2, wobei die Gradient-Norm durch die erste Ableitung der Funktion E = f(x) mittels der quantenchemischen Methode erhalten wird, mit E=Gesamtenergie der Vorstufe für den Übergangszustand und x=Kernkoordinaten des Moleküls in der Vorstufe für den Übergangszustand,
C4.1 wenn die Gradient-Norm C3 $\nabla$ $0 \leq \nabla \leq 0{,}07$ $E_h$ $a_0^{-1}$ beträgt, dann Fortführung des Verfahrens mit Schritt D1, oder
C4.2 wenn die Gradient-Norm C3 $\nabla > 0{,}07$ $E_h$ $a_0^{-1}$ beträgt, dann Wiederholung der Schritte C1 bis C3 bis eine Gradient-Norm C3 $0 \leq \nabla \leq 0{,}07$ $E_h$ $a_0^{-1}$ erhalten wird, wobei

(a) für den Fall, dass die Schritte C1 bis C3 ein Mal durchgeführt wurden, in Schritt C1 die Geometrie der Vorstufe für den Übergangszustand variiert wird, wenn der Wert ihrer Gradient-Norm C3 niedriger ist als der Wert der Gradient-Norm B3 der optimierten Startgeometrie, oder
(b) für den Fall dass die Schritte C1 bis C3 mehr als ein Mal durchgeführt wurden, in Schritt C1 die Geometrie der optimierten Startgeometrie oder derjenigen Vorstufe für den Übergangszustand aus den vorrangegangen Wiederholungen variiert wird, welche im Vergleich zu allen bisher erhaltenen Gradient-Normen C3 und B3 den niedrigsten Wert für die Gradient-Norm C3 oder B3 aufweist,

**D Ermittlung des Übergangszustandes**

D1 Relaxation der Vorstufe aus Schritt C4.1 oder der Vorstufe aus Schritt B4.1 mittels der quantenchemischen Methode und eines Pseudo-Newton-Raphson-Algorithmus, so dass der Übergangszustand erhalten wird,
D2 gegebenenfalls Ermittlung eines Gleichgewichtszustands durch Auslenkung des Übergangszustands, so dass ein ausgelenkter Übergangszustand erhalten wird und Relaxation des ausgelenkten Übergangszustands mittels der quantenchemischen Methode, so dass ein Gleichgewichtszustand erhalten wird.

In einer fünfzehnten Ausführungsform betrifft die Erfindung ein Computerprogramm, umfassend Befehle, die bei der Ausführung des Programms durch einen Computer diesen veranlassen, die folgenden Schritte eines Verfahrens durchzuführen:

**A Erzeugen einer Startgeometrie**

A1 Bereitstellung der dreidimensionalen Darstellung mindestens eines Moleküls im energetischen Grundzustand,
A2 Auswahl von mindestens einer Bindung des mindestens einen Moleküls und Auswahl einer Länge der Bindung, wobei die ausgewählte Länge nicht der Länge der Bindung im energetischen Grundzustand des Moleküls entspricht, so dass eine Startgeometrie für die chemische Reaktion erhalten wird,
A3 Dreidimensionale Darstellung der Startgeometrie in kartesischen und/oder internen Koordinaten,

**B Ermittlung einer optimierten Startgeometrie**

B1 Festlegung eines Funktionsraumes welcher die mindestens eine Bindung aus Schritt A2 und die durch diese Bindung verbundenen Atome umfasst,
B2 Geometrieoptimierung der Startgeometrie unter Zugrundelegung des in Schritt B1 ausgewählten Funktionsraums mittels einer quantenchemischen Methode und mit der Randbedingung, dass die in Schritt A2 ausgewählte Länge der mindestens einen Bindung konstant gehalten wird, so dass eine optimierte Startgeometrie erhalten wird,

B3 Ermittlung der Gradient-Norm B3 für die optimierte Startgeometrie, wobei die Gradient-Norm durch die erste Ableitung einer Funktion E = f(x) mittels der quantenchemischen Methode erhalten wird, mit E=Gesamtenergie der optimierten Startgeometrie und x=Kernkoordinaten des Moleküls in der optimierten Startgeometrie,
B4.1 wenn die Gradient-Norm B3 $\nabla$ $0 \leq \nabla \leq 0{,}07$ $E_h$ $a_0^{-1}$ beträgt, dann Einordnung der optimierten Startgeometrie als eine Vorstufe für den Übergangszustand der chemischen Reaktion und Fortführung des Verfahrens mit Schritt D1, oder
B4.2 wenn die Gradient-Norm B3 $\nabla > 0{,}07$ $E_h$ $a_0^{-1}$ beträgt, dann Ermittlung der Vorstufe für den Übergangszustand der chemischen Reaktion ausgehend von der optimierten Startgeometrie durch ein Verfahren umfassend die folgenden Schritte:

**C Ermittlung der Vorstufe für den Übergangszustand der chemischen Reaktion**

C1 Variation der optimierten Startgeometrie unter Anwendung eines Monte-Carlo-Algorithmus wobei
C1.1 wenigstens ein Atom aus dem in Schritt B1 ausgewählten Funktionsraum zufällig ausgewählt wird,
C1.2 ein Vektor für eine Auslenkung des in Schritt C1.1 gewählten Atoms zufällig ausgewählt wird,
C1.3 das in Schritt C1.1 ausgewählte Atom anhand des Vektors aus Schritt C1.2 aus seiner Position in der optimierten Startgeometrie ausgelenkt wird, so dass eine Vorstufe für den Übergangszustand der chemischen Reaktion erhalten wird,
C2 Geometrieoptimierung der Vorstufe für den Übergangszustand mittels der quantenchemischen Methode und mit der Randbedingung, dass die mindestens eine Bindung aus Schritt A2 die Länge hat, die in der Schritt C1.3 ermittelt wurde,
C3 Ermittlung der Gradient-Norm C3 für die Vorstufe für den Übergangszustand aus Schritt C2, wobei die Gradient-Norm durch die erste Ableitung der Funktion E = f(x) mittels der quantenchemischen Methode erhalten wird, mit E=Gesamtenergie der Vorstufe für den Übergangszustand und x=Kernkoordinaten des Moleküls in der Vorstufe für den Übergangszustand,

C4.1 wenn die Gradient-Norm C3 $\nabla$ $0 \leq \nabla \leq 0{,}07$ $E_h\,a_0^{-1}$ beträgt, dann Fortführung des Verfahrens mit Schritt D1, oder C4.2 wenn die Gradient-Norm C3 $\nabla > 0{,}07$ $E_h\,a_0^{-1}$ beträgt, dann Wiederholung der Schritte C1 bis C3 bis eine Gradient-Norm C3 $0 \leq \nabla \leq 0{,}07$ $E_h\,a_0^{-1}$ erhalten wird, wobei

(a) für den Fall, dass die Schritte C1 bis C3 ein Mal durchgeführt wurden, in Schritt C1 die Geometrie der Vorstufe für den Übergangszustand variiert wird, wenn der Wert ihrer Gradient-Norm C3 niedriger ist als der Wert der Gradient-Norm B3 der optimierten Startgeometrie, oder

(b) für den Fall dass die Schritte C1 bis C3 mehr als ein Mal durchgeführt wurden, in Schritt C1 die Geometrie der optimierten Startgeometrie oder derjenigen Vorstufe für den Übergangszustand aus den vorrangegangen Wiederholungen variiert wird, welche im Vergleich zu allen bisher erhaltenen Gradient-Normen C3 und B3 den niedrigsten Wert für die Gradient-Norm C3 oder B3 aufweist,

**D Ermittlung des Übergangszustandes**

D1 Relaxation der Vorstufe aus Schritt C4.1 oder der Vorstufe aus Schritt B4.1 mittels der quantenchemischen Methode und eines Pseudo-Newton-Raphson-Algorithmus, so dass der Übergangszustand erhalten wird, D2 gegebenenfalls Ermittlung eines Gleichgewichtszustands durch Auslenkung des Übergangszustands, so dass ein ausgelenkter Übergangszustand erhalten wird und Relaxation des ausgelenkten Übergangszustands mittels der quantenchemischen Methode, so dass ein Gleichgewichtszustand erhalten wird.

In einer sechzehnten Ausführungsform betrifft die Erfindung ein computerlesbares Speichermedium, umfassend Befehle, die bei der Ausführung durch einen Computer diesen veranlassen, die folgenden Schritte eines Verfahrens durchzuführen:

**A Erzeugen einer Startgeometrie**

A1 Bereitstellung der dreidimensionalen Darstellung mindestens eines Moleküls im energetischen Grundzustand, A2 Auswahl von mindestens einer Bindung des mindestens einen Moleküls und Auswahl einer Länge der Bindung, wobei die ausgewählte Länge nicht der Länge der Bindung im energetischen Grundzustand des Moleküls entspricht, so dass eine Startgeometrie für die chemische Reaktion erhalten wird, A3 Dreidimensionale Darstellung der Startgeometrie in kartesischen und/oder internen Koordinaten,

**B Ermittlung einer optimierten Startgeometrie**

B1 Festlegung eines Funktionsraumes welcher die mindestens eine Bindung aus Schritt A2 und die durch diese Bindung verbundenen Atome umfasst, B2 Geometrieoptimierung der Startgeometrie unter Zugrundelegung des in Schritt B1 ausgewählten Funktionsraums mittels einer quantenchemischen Methode und mit der Randbedingung, dass die in Schritt A2 ausgewählte Länge der mindestens einen Bindung konstant gehalten wird, so dass eine optimierte Startgeometrie erhalten wird,

B3 Ermittlung der Gradient-Norm B3 für die optimierte Startgeometrie, wobei die Gradient-Norm durch die erste Ableitung einer Funktion E = f(x) mittels der quantenchemischen Methode erhalten wird, mit E=Gesamtenergie der optimierten Startgeometrie und x=Kernkoordinaten des Moleküls in der optimierten Startgeometrie, B4.1 wenn die Gradient-Norm B3 $\nabla$ $0 \leq \nabla \leq 0{,}07$ $E_h\,a_0^{-1}$ beträgt, dann Einordnung der optimierten Startgeometrie als eine Vorstufe für den Übergangszustand der chemischen Reaktion und Fortführung des Verfahrens mit Schritt D1, oder B4.2 wenn die Gradient-Norm B3 $\nabla > 0{,}07$ $E_h\,a_0^{-1}$ beträgt, dann Ermittlung der Vorstufe für den Übergangszustand der chemischen Reaktion ausgehend von der optimierten Startgeometrie durch ein Verfahren umfassend die folgenden Schritte:

**C Ermittlung der Vorstufe für den Übergangszustand der chemischen Reaktion**

C1 Variation der optimierten Startgeometrie unter Anwendung eines Monte-Carlo-Algorithmus wobei
C1.1 wenigstens ein Atom aus dem in Schritt B1 ausgewählten Funktionsraum zufällig ausgewählt wird,
C1.2 ein Vektor für eine Auslenkung des in Schritt C1.1 gewählten Atoms zufällig ausgewählt wird,
C1.3 das in Schritt C1.1 ausgewählte Atom anhand des Vektors aus Schritt C1.2 aus seiner Position in der optimierten Startgeometrie ausgelenkt wird, so dass eine Vorstufe für den Übergangszustand der chemischen Reaktion erhalten wird,
C2 Geometrieoptimierung der Vorstufe für den Übergangszustand mittels der quantenchemischen Methode und mit der Randbedingung, dass die mindestens eine Bindung aus Schritt A2 die Länge hat, die in der Schritt C1.3 ermittelt wurde,
C3 Ermittlung der Gradient-Norm C3 für die Vorstufe für den Übergangszustand aus Schritt C2, wobei die Gradient-Norm durch die erste Ableitung der Funktion E = f(x) mittels der quantenchemischen Methode erhalten wird, mit E=Gesamtenergie der Vorstufe für den Übergangszustand und x=Kernkoordinaten des Moleküls in der Vorstufe für den Übergangszustand,
C4.1 wenn die Gradient-Norm C3 $\nabla$ $0 \leq \nabla \leq 0{,}07$ $E_h\, a_0^{-1}$ beträgt, dann Fortführung des Verfahrens mit Schritt D1, oder
C4.2 wenn die Gradient-Norm C3 $\nabla > 0{,}07$ $E_h\, a_0^{-1}$ beträgt, dann Wiederholung der Schritte C1 bis C3 bis eine Gradient-Norm C3 $0 \leq \nabla \leq 0{,}07$ $E_h\, a_0^{-1}$ erhalten wird, wobei

(a) für den Fall, dass die Schritte C1 bis C3 ein Mal durchgeführt wurden, in Schritt C1 die Geometrie der Vorstufe für den Übergangszustand variiert wird, wenn der Wert ihrer Gradient-Norm C3 niedriger ist als der Wert der Gradient-Norm B3 der optimierten Startgeometrie, oder

(b)für den Fall dass die Schritte C1 bis C3 mehr als ein Mal durchgeführt wurden, in Schritt C1 die Geometrie der optimierten Startgeometrie oder derjenigen Vorstufe für den Übergangszustand aus den vorrangegangen Wiederholungen variiert wird, welche im Vergleich zu allen bisher erhaltenen Gradient-Normen C3 und B3 den niedrigsten Wert für die Gradient-Norm C3 oder B3 aufweist,

**D Ermittlung des Übergangszustandes**

D1 Relaxation der Vorstufe aus Schritt C4.1 oder der Vorstufe aus Schritt B4.1 mittels der quantenchemischen Methode und eines Pseudo-Newton-Raphson-Algorithmus, so dass der Übergangszustand erhalten wird,
D2 gegebenenfalls Ermittlung eines Gleichgewichtszustands durch Auslenkung des Übergangszustands, so dass ein ausgelenkter Übergangszustand erhalten wird und Relaxation des ausgelenkten Übergangszustands mittels der quantenchemischen Methode, so dass ein Gleichgewichtszustand erhalten wird.

In einer siebzehnten Ausführungsform betrifft die Erfindung die Verwendung eines Computerprogramms nach Ausführungsform 15 oder eines computerlesbaren Speichermediums nach Ausführungsform 18 zur Bewertung von Übergangszuständen einer chemischen Reaktion, insbesondere einer Polymersynthese.
In einer achtzehnten Ausführungsform betrifft die Erfindung Verfahren gemäß einer der ersten bis dreizehnten Ausführungsform , dadurch gekennzeichnet, dass Information über den gemäß Schritt D.1 ermittelten Übergangszustand und/oder der gemäß Schritt D.2 ermittelten Gleichgewichtszustand einem Benutzer mitgeteilt wird.
In einer neunzehnten Ausführungsform betrifft die Erfindung Verfahren gemäß einer der ersten bis dreizehnten Ausführungsform, dadurch gekennzeichnet, dass Information über den gemäß Schritt D.1 ermittelten Übergangszustand und/oder der gemäß Schritt D.2 ermittelten Gleichgewichtszustand durch einen Benutzer empfangen wird.
In einer zwanzigsten Ausführungsform betrifft die Erfindung Verfahren gemäß einer der neunten bis dreizehnten Ausführungsform, dadurch gekennzeichnet, dass nach Schritt D.1 und/oder nach Schritt D.2 das Molekül I synthetisiert wird.
In einer einundzwanzigsten Ausführungsform betrifft die Erfindung Verfahren gemäß einer der neunten bis dreizehnten Ausführungsform, dadurch gekennzeichnet, dass nach Schritt D.1 und/oder nach Schritt D.2 unter eine chemische Reaktion mit dem Molekül I als Katalysator durchgeführt wird.
In einer zweiundzwanzigsten Ausführungsform betrifft die Erfindung Verfahren gemäß einer der neunten bis dreizehnten oder einundzwanzigsten Ausführungsform dadurch gekennzeichnet, dass nach Schritt D.1 und/oder nach Schritt D.2 unter eine chemische Reaktion mit dem Molekül II als Reaktionspartner durchgeführt wird.

Anhand der nachfolgenden Beispiele soll die Erfindung verdeutlicht werden, ohne jedoch darauf beschränkt zu werden.

**A Beispiele zur Ermittlung einer vor optimierten Startgeometrie**

Das erfindungsgemäße Verfahren und ein herkömmliches Verfahren wurden auf verschiedene Reaktionen angewandt. Dazu wurde zunächst für jede Reaktion mit Hilfe eines kommerziell erhältlichen Programms zur Generierung und Visualisierung von dreidimensionalen Strukturen chemischer Moleküle eine Startgeometrie des unbekannten, gesuchten Übergangszustandes gezeichnet. Dabei wurden die Abstände zwischen den Atomen bzw. der Bindungen geschätzt, in dem die typischen Bindungslängen im Grundzustand um 10-90% verlängert wurden.

Im Fall des in Tabelle A2 gezeigten Ausführungsbeispiels wurde beispielsweise, gemäß Verfahrensschritt A1, ein Ethen Molekül zusammen mit einem Butadienmolekül gezeichnet, mittels eines Molekularvisualiserungsprogramms wie z. B. TMoleX oder Avogadro. Gemäß Verfahrensschritt A2 wurden die zwei zum Cyclohexen führenden Verbindungsachsen der terminalen Doppelbindungskohlenstoffe auf eine Distanz von 179,7 pm und 164,1 pm gebracht und die so erzeugte Molekülgeometrie gemäß Verfahrensschritt A3 in einer Darstellung kartesischer Koordinaten gespeichert, mittels eines Molekularvisualiserungsprogramms, wie z. B. TMoleX oder Avogadro. Die an den so angeordneten Bindungsabständen beteiligten Atome wurden anschließend zur Definition des Funktionsraumes gemäß Verfahrensschritt B1 verwendet.

In Vergleichsversuchen wurde diese Startgeometrie einer Geometrieoptimierung mit einer Quasi-Newton- Raphson-Methode unterzogen, wie sie im Stand der Technik verwendet wird. Hierzu wurden zunächst die an der Bindungsdissoziation beteiligten Bindungsabstände konstant gehalten und eine Voroptimierung durchgeführt. Anschließend wurde die Quasi-Newton-Raphson-Methode angewendet. Dabei konnte kein Übergangszustand lokalisiert werden. Eine Wiederholung des letzten Schritts bringt keine Veränderung des Ergebnisses, da es sich um eine deterministische Methode handelt.

In den erfindungsgemäßen Versuchen wurde auf dieselbe Startgeometrie das erfindungsgemäße Verfahren angewendet. Dazu wurde zunächst die Voroptimierung mit der erfindungsgemäßen Monte-Carlo-Methode vorgenommen. Die erfindungsgemäße Monte Carlo-Methode variiert die festgelegten Bindungslängen mit Dissoziationscharakter nach Zufallszahlen und führt anschließend Geometrieoptimierungen mit konstant gehaltenen Bindungsabständen durch. Diese Prozedur wird iterativ durchlaufen, bis die erhaltene Gradient-Norm unter einen festgelegten Wert minimiert ist, wodurch eine ausreichende Nähe zu einem stationären Punkt indiziert wird. Pro untersuchter Reaktion wurden mit dem erfindungsgemäßen Monte-Carlo-Verfahren 10 optimierte Startgeometrien mit dem jeweils gleichen Wert für die Gradient-Norm als Ausgangspunkt für die anschließende Geometrieoptimierung mittels Quasi-Newton-Raphson gewählt.

Die Berechnungen und die Erfolgsquoten, d.h. die Anzahl der Übergangszustände, die auf einer Grundlage von jeweils zehn optimierten Startgeometrien ermittelt werden konnte, sind in Tabellen A1 bis A5 dargestellt.

Alle quantenmechanischen Berechnungen wurden dabei mit dem Programmpaket TURBOMOLE durchgeführt. Die verwendete Dichtefunktionaltheoretische Methode war das TPSS-Dichtefunktional mit einem def2-SVP-Basissatz, so wie standardmäßig im Turbomole-Programmpaket implementiert. Dazu wurde als Computer ein Intel Xeon E5-2667v4 mit 16 Core-Prozessoren mit einer Taktfrequenz von 3.20GHz und 25MB Zwischenspeicher mit einem RAM Speicher von 128 GB DDR4 2400 rg ECC.

Unter Verwendung dieses Rechners konnten mit dem erfindungsgemäßen Verfahren innerhalb von höchstens 2 Stunden für 10 unterschiedliche, manuell ausgewählte Startgeometrien einer Reaktion verschiedene Übergangszustände für diese Reaktion berechnet werden. Dabei konnte das Verfahren von dem Rechner auch ohne Kontrolle durch einen Mitarbeiter, z.B. über Nacht, durchgeführt werden.

Mit herkömmlichen Verfahren, bei denen die Startgeometrie manuell, d.h. ohne ein computerimplementiertes Verfahren, variiert wird, wäre ein Zeitaufwand von 2 bis 3 Tagen pro Startgeometrie erforderlich bis eine Geometrie gefunden worden wäre , auf die ein Pseudo-Newton-Raphson-Verfahren angewendet werden kann, d.h. erst nach 2 bis 3 könnte überhaupt versucht werden einen Übergangszustand zu berechnen.

In der nachfolgenden Tabelle A.1 ist zusammengefasst, wie oft Schritt B, bzw. die Schritte B und C, durchgeführt wurden

- für alle optimierten Startgeometrien pro untersuchter Reaktion, bis eine Vorstufe für den Übergangszustand erhalten wurde oder das Verfahren abgebrochen wurde, weil keine Vorstufe für den Übergangszustand erhalten wurde (Zeile 1), oder
- für diejenigen optimierten Startgeometrien, für die ein Übergangszustand ermittelt werden konnte (Zeile 2).

**Tabelle A.1: Statistische Auswertung**

| Zeile | Beispiele | Tab. 2 | Tab. 3 | Tab. 4 | Tab. 5 |
|---|---|---|---|---|---|
| 1 | Durchschnittliche Anzahl der Durchläufe von Schritt C des erfindungsgemäßen Verfahrens für alle optimierten Startgeometrien einer Reaktion | 6,6 | 9,7 | 8,2 | 9,2 |
| 2 | Durchschnittliche Anzahl der Durchläufe von Schritt C des erfindungsgemäßen Verfahrens für diejenigen optimierten Startgeometrien, für die ein Übergangszustand ermittelt werden konnte. | 5,5 | 12,3 | 8,2 | 9,2 |
| 3 | Prozent der jeweiligen optimierten Startgeometrien eines Versuchs für die mit dem erfindungsgemäßen Verfahren ein Übergangszustand ermittelt werden konnte | 80 | 30 | 100 | 100 |

Für die Bromierung von Ethen, in der folgenden Tabelle A3 dargestellt ist es relativ schwer einen Übergangszustand zu berechnen. Dies liegt sehr wahrscheinlich daran, dass der Sattelpunkt eine sehr spitze Form aufweist.

**Tabelle A.2: Cycloaddition von 1,3-Butadien und Ethen**

| Beispiel | Jede Spalte zeigt jeweils eine andere optimierte Startgeometrie | | | | | | | | | |
|---|---|---|---|---|---|---|---|---|---|---|
| | 2.1 | 2.2 | 2.3 | 2.4 | 2.5 | 2.6 | 2.7 | 2.8 | 2.9 | 2.10 |
| Gradient-Norm B3 $[E_h\ a_0^{-1}]$ der jeweiligen optimierten Startgeometrie | 0.101578 | 0.101578 | 0.101578 | 0.101578 | 0.101578 | 0.101578 | 0.101578 | 0.101578 | 0.101578 | 0.101578 |
| Gradient-Norm C3 $[E_h\ a_0^{-1}]$, wobei jede Zeile die Gradient Norm einer anderen Vorstufe für den Übergangszustand jeweils basierend auf der optimierten Startgeometrie der ersten Zeile darstellt | 0.063475 | 0.112442 | 0.027745 | 0.094052 | 0.125426 | 0.086226 | 0.081093 | 0.107781 | 0.087146 | 0.125814 |
| | 0.074802 | 0.074597 | | 0.029719 | 0.091124 | 0.088933 | 0.095923 | 0.111804 | 0.159795 | 0.041008 |
| | 0.357504 | 0.090029 | | | 0.065701 | 0.009156 | 0.206321 | 0.070290 | 0.110302 | 0.667969 |
| | 0.090818 | 0.390142 | | | 0.053928 | | 0.063935 | 0.072940 | 0.118519 | 0.194443 |
| | 0.111428 | 0.092513 | | | 0.012537 | | 0.012706 | 0.056430 | 2.170.497 | 0.013739 |
| | 1.404.968 | 0.067496 | | | | | | 0.061099 | 0.071430 | |
| | 0.111726 | 0.029182 | | | | | | 0.025372 | 0.085191 | |
| | 0.102065 | | | | | | | | 0.024142 | |
| | 0.077888 | | | | | | | | | |
| | 0.085817 | | | | | | | | | |
| | 0.057883 | | | | | | | | | |
| | 0.194193 | | | | | | | | | |
| | 0.037484 | | | | | | | | | |
| Erhalt eines Übergangszustands | Nein | Nein | Ja | Ja | Ja | Ja | Ja | Ja | Ja | Ja |

EP 3 867 913 B1

In Beispiel 2.1 wurde die Wiederholung des Schritts C des erfindungsgemäßen Verfahrens nach 13 Durchläufen abgebrochen, weil keine Vorstufe für den Übergangszustand ermittelt werden konnte, die als Ausgangspunkt für die Ermittlung eines Übergangszustands mit Hilfe eines Pseudo-Newton-Raphson-Algorithmus nach Schritt D1 geeignet war.

**Tabelle A.3: Bromierung von Ethen zu 1,2 Bromethan**

| Beispiel | Jede Spalte zeigt jeweils eine andere optimierte Startgeometrie | | | | | | | | | |
|---|---|---|---|---|---|---|---|---|---|---|
|  | 3.1 | 3.2 | 3.3 | 3.4 | 3.5 | 3.6 | 3.7 | 3.8 | 3.9 | 3.10 |
| Gradient-Norm B3 $[E_h\,a_0^{-1}]$ der jeweiligen optimierten Startgeometrie | 0.056813 | 0.056813 | 0.056813 | 0.056813 | 0.056813 | 0.056813 | 0.056813 | 0.056813 | 0.056813 | 0.056813 |
| Gradient-Norm C3 $[E_h\,a_0^{-1}]$, wobei jede Zeile die Gradient Norm einer anderen Vorstufe für den Übergangszustand jeweils basierend auf der optimierten Startgeometrie der ersten Zeile darstellt | 0.058529 | 0.064647 | 0.064082 | 0.048314 | 0.031738 | 0.058399 | 0.051681 | 0.058077 | 0.127864 | 0.231416 |
|  | 0.251325 | 0.061090 | 0.152230 | 0.137252 |  | 0.059887 | 0.044425 | 0.060593 | 0.232556 | 0.056812 |
|  | 0.064038 | 0.107419 | 0.051544 | 0.056970 |  | 0.060656 | 0.066049 | 0.053602 | 0.057414 | 0.063343 |
|  | 0.056935 | 0.065046 | 0.099173 | 0.048362 |  | 0.284270 | 0.081247 | 0.070952 | 0.044737 | 0.065012 |
|  | 0.059306 | 0.062879 | 0.112987 | 0.056837 |  | 0.058757 | 0.057149 | 0.099214 | 0.035028 | 0.256491 |
|  | 0.030828 | 0.442817 | 0.050282 | 0.135475 |  | 0.060478 | 0.036297 | 0.055646 |  | 0.045294 |
|  |  | 0.058038 | 0.070672 | 0.063971 |  | 0.062872 |  | 0.046495 |  | 0.056140 |
|  |  | 0.054012 | 0.061423 | 0.045588 |  | 0.049047 |  | 0.046513 |  | 0.045318 |
|  |  | 0.033991 |  | 0.090480 |  | 0.054034 |  | 0.050048 |  | 0.055720 |
|  |  |  |  | 0.067227 |  | 0.046588 |  | 0.048978 |  | 0.168430 |
|  |  |  |  | 0.044884 |  | 0.055280 |  | 0.048104 |  | 0.059298 |
|  |  |  |  | 0.057821 |  |  |  | 0.056875 |  | 8.418.276 |
|  |  |  |  |  |  |  |  | 0.089174 |  |  |
|  |  |  |  |  |  |  |  | 0.057857 |  |  |
|  |  |  |  |  |  |  |  | 2.128.698 |  |  |
|  |  |  |  |  |  |  |  | 0.085683 |  |  |
|  |  |  |  |  |  |  |  | 0.037714 |  |  |
| Erhalt eines Übergangszustands | Ja | Nein | Nein | Nein | Nein | Ja | Nein | Ja | Nein | Nein |

Die gezeigten Werte umfassen auch statistische Extremalwerte, z.B. in Spalten 3.8 und 3.10.

**Tabelle A.4: Decarboxylierung von Acetessigsäure**

| | Jede Spalte zeigt jeweils eine andere optimierte Startgeometrie | | | | | | | | | |
|---|---|---|---|---|---|---|---|---|---|---|
| Beispiel | 4.1 | 4.2 | 4.3 | 4.4 | 4.5 | 4.6 | 4.7 | 4.8 | 4.9 | 4.10 |
| Gradient-Norm B3 [$E_h\,a_0^{-1}$] der jeweiligen optimierten Startgeometrie | 0.055244 | 0.055145 | 0.055285 | 0.055128 | 0.055106 | 0.055109 | 0.055218 | 0.055265 | 0.055413 | 0.055211 |
| Gradient-Norm C3 [$E_h\,a_0^{-1}$], wobei jede Zeile die Gradient Norm einer anderen Vorstufe für den Übergangszustand jeweils basierend auf der optimierten Startgeometrie der ersten Zeile darstellt | 0.049610 | 0.050850 | 0.057335 | 0.077653 | 0.060983 | 0.047224 | 0.050552 | 0.057350 | 0.058160 | 0.069370 |
| | 0.049458 | 0.062125 | 0.080891 | 0.058727 | 0.065141 | 0.067408 | 0.057388 | 0.621287 | 0.022109 | 0.082815 |
| | 0.019246 | 0.039781 | 0.045796 | 0.043377 | 0.059457 | 0.112063 | 0.034994 | 0.052275 | | 0.087075 |
| | | | 0.053588 | 0.071861 | 0.053003 | 0.051080 | | 0.056791 | | 0.113501 |
| | | | 0.066665 | 0.069440 | 0.051556 | 0.055590 | | 0.072648 | | 0.051053 |
| | | | 0.020932 | 0.077257 | 0.062801 | 0.073973 | | 0.045846 | | 0.048086 |
| | | | | 0.045192 | 0.047389 | 0.087384 | | 0.047361 | | 0.051334 |
| | | | | 0.053762 | 0.113882 | 0.066149 | | 0.015354 | | 0.010003 |
| | | | | 0.057251 | 0.069916 | 0.056958 | | | | |
| | | | | 0.089196 | 0.061307 | 0.083602 | | | | |
| | | | | 0.022387 | 0.045287 | 0.044368 | | | | |
| | | | | | 0.099284 | 0.042818 | | | | |
| | | | | | 0.326467 | 0.054379 | | | | |
| | | | | | 0.053152 | 0.030123 | | | | |
| Erhalt eines Übergangszustands | Ja | Ja | Ja | Ja | Ja | Ja | Ja | Ja | Ja | Ja |

**Tabelle A.5: Reduktion von Aceton zu Isobuten**

| Beispiel | 5.1 | 5.2 | 5.3 | 5.4 | 5.5 | 5.6 | 5.7 | 5.8 | 5.9 | 5.10 |
|---|---|---|---|---|---|---|---|---|---|---|
| | Jede Spalte zeigt jeweils eine andere optimierte Startgeometrie | | | | | | | | | |
| Gradient-Norm B3 [$E_h$ $a_0^{-1}$] der jeweiligen optimierten Startgeometrie | 0,070525 | 0,070525 | 0,070525 | 0,070525 | 0,070525 | 0,070525 | 0,070525 | 0,070525 | 0,070525 | 0,070525 |
| Gradient-Norm C3 [$E_h$ $a_0^{-1}$], wobei jede Zeile die Gradient Norm einer anderen Vorstufe für den Übergangszustand jeweils basierend auf der optimierten Startgeometrie der ersten Zeile darstellt | 0,053417 | 0,039139 | 0,080632 | 0,051345 | 0,066935 | 0,067211 | 0,07307 | 0,070434 | 0,070324 | 0,086278 |
| | 0,032317 | | 0,086216 | 0,090356 | 0,047545 | 0,068587 | 0,071768 | 0,064323 | 0,071679 | 0,057452 |
| | | | 0,055797 | 0,054938 | 0,026039 | 0,063053 | 0,054758 | 0,066577 | 0,052235 | 0,019717 |
| | | | 0,065289 | 0,0546 | | 0,056315 | 0,059952 | 0,062511 | 0,061696 | |
| | | | 0,052484 | 0,058829 | | 0,048515 | 0,061317 | 0,060599 | 0,036104 | |
| | | | 0,043561 | 0,054373 | | 0,045782 | 0,069163 | 0,171984 | | |
| | | | 0,057211 | 0,0659 | | 0,031459 | 0,05429 | 0,060208 | | |
| | | | 0,087309 | 0,044661 | | | 0,044025 | 0,066551 | | |
| | | | 0,044804 | 0,283666 | | | 0,044025 | 0,067845 | | |
| | | | 0,053187 | 0,054703 | | | 0,045235 | 0,057384 | | |
| | | | 0,045268 | 0,043981 | | | 0,044025 | 0,06995 | | |
| | | | 0,057168 | 0,039904 | | | | 0,065572 | | |
| | | | 0,053825 | | | | | 0,060108 | | |
| | | | 0,056278 | | | | | 0,060921 | | |
| | | | 0,084044 | | | | | 0,060597 | | |
| | | | 0,043251 | | | | | 0,045019 | | |
| | | | 0,043252 | | | | | 0,057638 | | |
| | | | 0,045823 | | | | | 0,065807 | | |
| | | | 0,039945 | | | | | 0,033495 | | |
| Erhalt eines Übergangszustands | Ja | Ja | Ja | Ja | Ja | Ja | Ja | Ja | Ja | Ja |

**B Beispiele zur Ermittlung eines Katalysators für eine chemische Reaktion**

In den folgenden Beispielen wurde mit Hilfe des erfindungsgemäßen Verfahrens untersucht, welcher der bekannten Katalysatoren 1,4-Diazabicyclo[2.2.2]octan (DABCO) oder N,N-Dimethylannillin (DMA) sich besser für die Hydrolyse von unterschiedlichen Isocyanaten eignet. Mit dieser Reaktion kann bei der Herstellung von Polyurethanschaumstoffen die Reaktionsmischung durch den Eintrag des entstandenen Gases aufgetrieben werden, so dass ein Schaum erhalten wird.

In den Beispielen wurde zunächst mit Hilfe des erfindungsgemäßen Verfahrens die Aktivierungsenergien für die betreffende Reaktion von Isocyanat und Wasser berechnet und die erhaltenen Werte dann mit den Ergebnissen von Laborversuchen der zuvor berechneten Reaktionen verglichen. Als Maßstab für die Aktivität der getesteten Katalysatoren wurde bei den Laborversuchen die Reaktionstemperatur herangezogen oberhalb derer die Katalysatoren Aktivität zeigten. Ob die Katalysatoren Aktivität zeigten wurde im Laborexperiment anhand der Freisetzung von Gasen und dem Aufschäumen der Reaktionsmischung festgestellt.

Die quantenmechanischen Berechnungen wurden mit dem Programmpaket Turbomole durchgeführt. Die verwendete Dichtefunktionaltheoretische Methode war das TPSS-Dichtefunktional mit einem def2-SVP-Basissatz, so wie standardmäßig im Turbomole-Programmpaket implementiert. Dazu wurde als Computer ein Intel Xeon E5-2667v4 mit 16 Core-Prozessoren mit einer Taktfrequenz von 3.20GHz und 25MB Zwischenspeicher mit einem RAM Speicher von 128 GB DDR4 2400 rg ECC.

Es wurden pro untersuchter Reaktion eine Startgeometrie als Ausgangspunkt für die Ermittlung eines Übergangszustandes gewählt. Der Zeitaufwand für die Berechnung der jeweiligen Übergangszustände betrug bis zu 8 Stunden.

Die Laborversuche wurden wie folgt durchgeführt:

In einem Reagenzglas mit Magnetrührer wurden 0,1 Äquivalente Katalysator, 1,0 Äquivalente Polyethylenglykol und 1,0 Äquivalente Wasser vorgelegt. Der Inhalt des Reagenzglas wurde gründlich durchmischt und unter Rühren 1,0 Äquivalente des betreffenden Isocyanats zugesetzt. Die Temperatur des Gemischs wurde mit Hilfe eines Ölbads mit Thermostat langsam erhöht, bis eine deutliche Gasentwicklung beobachtet werden konnte.

**Tabelle B.1: Molmassen und Stoffmengen der eingesetzten Substanzen.**

| Komponente | M [g/mol] | n [mol] | m [mg] | Äq | d [g/ml] | V [ml] |
|---|---|---|---|---|---|---|
| DABCO | 112,17 | 1,722 | 193 | 0,1 | - | - |
| DMA | 121,19 | 1,722 | 208 | 0,1 | 0,96 | 0,22 |
| TDI | 147,20 | 1,722 | 3000 | 1,0 | 1,22 | 2,46 |
| IPDI | 222,29 | 1,722 | 3828 | 1,0 | 1,06 | 3,61 |
| $H_2O$ | 18,02 | 1,722 | 310 | 1,0 | 1,00 | 0,31 |
| PEG | 400 | 0,861 | 3444 | 0,5 | 1,128 | 3,05 |

**Beispiel B.1**: **1,4-Diazabicyclo[2.2.2]octan vs N,N-Dimethylannillin als Katalysatoren für die Hydrolyse von 1,4-Toluyldiisocyanat**

Zur Evaluierung der katalytischen Aktivität von 1,4-Diazabicyclo[2.2.2]octan zur Katalysierung der Hydrolyse von 1,4-Toluyldiisocyanat mit Wasser wurde die Anlagerung von Wasser an das Isocyanat-Molekül mit der erfindungsgemäßen Methode zur Berechnung von Übergangszuständen berechnet. Für die mit dem erfindungsgemäßen Verfahren ermittelte Geometrie des Übergangszustands wurde eine Aktivierungsenergie von 12,8 Kcal/mol berechnet. Dieselbe Prozedur wurde für N,N-Dimethylannillin (s. Tabelle B.2) wiederholt und eine Aktivierungsenergie von 17.1 Kcal/mol berechnet.

Unter den oben beschriebenen experimentellen Bedingungen zeigte die Umsetzung von 1,4-Toluyldiisocyanat mit Wasser in Gegenwart von 1,4-Diazabicyclo[2.2.2]octan als Katalysator eine signifikante Reaktion bei 20°C. Unter identischen Bedingungen wurde N,N-Dimethylannillin als Katalysator getestet. Hier war eine Reaktionstemperatur von 39°C notwendig um eine beobachtbare chemische Reaktion zu herbei zu führen.

**Tabelle B.2: Zusammenstellung experimenteller und simulierter Daten**

| Isocyanat | Katalysator | Aktivierungsenergie Simuliert [Kcal/mol] | Temperatur bei der eine Aktivität des Katalysators im Laborversuch zu beobachten war [°C] | Gradientnorm der optimierten Startgeometrie $[E_h\ a_0^{-1}]$ | Gradientnorm des Übergangszustands $[E_h\ a_0^{-1}]$ |
|---|---|---|---|---|---|
| TDI | DABCO | 12,8 | 20 | 0,067 | 0,042 |
| TDI | DMA | 17,1 | 39 | 0,068 | 0,030 |

Dieser Vergleich zeigt, dass mit Hilfe der erfindungsgemäßen Methode die Molekülgeometrie eines Übergangszustands einer chemischen Reaktion so präzise berechnet werden kann, dass die Aktivierungsenergie, die für diese Molekülgeometrie berechnet wird, eine zutreffende Aussage über die Kinetik der chemischen Reaktion erlaubt. Die auf der Grundlage des erfindungsgemäßen Verfahrens berechnete Aktivierungsenergie für DABCO war deutlich niedriger als diejenige für DMA. Aus den berechneten Ergebnissen kann der Schluss gezogen werden, dass DABCO als Katalysator für die Hydrolyse von 1,4-Toluyldiisocyanat einen Übergangszustand ermöglicht der eine niedrigere Energie aufweist als bei der Verwendung von DMA als Katalysator und dass folglich bei der Durchführung der Reaktion mit DABCO als Katalysator weniger oder keine Energie (ausgehend von Raumtemperatur) zugeführt werden muss, damit die Reaktion abläuft und Produkte erhalten werden.

Diese Schlüsse aus den Berechnungen spiegeln sich in den Ergebnissen der Laborversuche wieder. Während die Hydrolyse von 1,4-Toluyldiisocyanat mit DABCO als Katalysator bereits bei einer Reaktionstemperatur von 20 °C ablief, musste bei der Verwendung von DMA Energie zugeführt werden, und die Reaktion lief erst bei 39 °C ab.

**Beispiel B.2: 1,4-Diazabicyclo[2.2.2]octan vs N,N-Dimethylannillin als Katalysatoren für die Hydrolyse von Isophorondiisocyanat**

Zur Evaluierung der Katalytischen Aktivität von 1,4-Diazabicyclo[2.2.2]octan zur Katalysierung der Hydrolyse von Isophorondiisocyanat mit Wasser wurde die Anlagerung von Wasser an das Isocyanat-Molekül mit der erfindungsgemäßen Methode zur Berechnung von Übergangszuständen berechnet. Für die mit dem erfindungsgemäßen Verfahren ermittelte Geometrie des Übergangszustands wurde eine Aktivierungsenergie von 16,3 Kcal/mol berechnet.

Die gleiche Prozedur wurde für N,N-Dimethylannillin (s. Tabelle B.3) wiederholt und eine Aktivierungsenergie von 19,9 Kcal/mol berechnet.

Unter den beschriebenen experimentellen Bedingungen zeigte die Umsetzung von Isophorondiisocyanat mit Wasser in Gegenwart von 1,4-Diazabicyclo[2.2.2]octan als Katalysator eine signifikante Reaktion bei 31°C. Unter identischen Bedingungen wurde N,N-Dimethylannillin als Katalysator getestet. Hier war eine Reaktionstemperatur von 116°C notwendig um eine beobachtbare chemische Reaktion zu herbei zu führen.

**Tabelle B.3: Zusammenstellung experimenteller und simulierter Daten**

| Isocyanat | Katalysator | Aktivierungsenergie Simuliert [Kcal/mol] | Temperatur bei der eine Aktivität des Katalysators im Laborversuch zu beobachten war [°C] | Gradientnorm der optimierten Startgeometrie $[E_h\ a_0^{-1}]$ | Gradientnorm des Übergangszustands $[E_h\ a_0^{-1}]$ |
|---|---|---|---|---|---|
| IPDI | DABCO | 16,3 | 31 | 0,067 | 0,043 |
| IPDI | DMA | 19,9 | 116 | 0,067 | 0,045 |

Dieser Vergleich zeigt, dass mit Hilfe der erfindungsgemäßen Methode die Molekülgeometrie eines Übergangszustands einer chemischen Reaktion mit sehr wenig Benutzeraufwand und so präzise berechnet werden kann, dass die Aktivierungsenergie, die für diese Molekülgeometrie berechnet wird, eine zutreffende Aussage über die Kinetik der chemischen Reaktion erlaubt. Die auf der Grundlage des erfindungsgemäßen Verfahrens berechnete Aktivierungsenergie für DABCO war deutlich niedriger als diejenige für DMA. Aus den berechneten Ergebnissen kann der Schluss gezogen werden, dass DABCO als Katalysator für die Hydrolyse von 1,4-Toluyldiisocyanat einen Übergangszustand ermöglicht der eine niedrigere Energie aufweist als bei der Verwendung von DMA als Katalysator und dass folglich bei der Durchführung der Reaktion mit DABCO als Katalysator weniger oder keine Energie (ausgehend von Raumtemperatur) zugeführt werden muss, damit die Reaktion abläuft und Produkte erhalten werden.

Diese Schlüsse aus den Berechnungen spiegeln sich in den Ergebnissen der Laborversuche wieder. Während die Hydrolyse von 1,4-Toluyldiisocyanat mit DABCO als Katalysator bereits bei einer Reaktionstemperatur bei 31 °C ablief, musste bei der Verwendung von DMA deutlich mehr Energie zugeführt werden, und die Reaktion lief erst bei 116 °C ab.

## Patentansprüche

1. Computerimplementiertes Verfahren zur Berechnung von Übergangszuständen einer chemischen Reaktion umfassend die Schritte

   **A Erzeugen einer Startgeometrie**
   A1 Bereitstellung der dreidimensionalen Darstellung mindestens eines Moleküls im energetischen Grundzustand,
   A2 Auswahl von mindestens einer Bindung des mindestens einen Moleküls und Auswahl einer Länge der Bindung, wobei die ausgewählte Länge nicht der Länge der Bindung im energetischen Grundzustand des Moleküls entspricht, so dass eine Startgeometrie für die chemische Reaktion erhalten wird,
   A3 Dreidimensionale Darstellung der Startgeometrie in kartesischen und/oder internen Koordinaten,
   **B Ermittlung einer optimierten Startgeometrie**
   B1 Festlegung eines Funktionsraumes welcher die mindestens eine Bindung aus Schritt A2 und die durch diese Bindung verbundenen Atome umfasst,
   B2 Geometrieoptimierung der Startgeometrie unter Zugrundelegung des in Schritt B1 ausgewählten Funktionsraums mittels einer quantenchemischen Methode und mit der Randbedingung, dass die in Schritt A2 ausgewählte Länge der mindestens einen Bindung konstant gehalten wird, so dass eine optimierte Startgeometrie erhalten wird,
   B3 Ermittlung der Gradient-Norm B3 für die optimierte Startgeometrie, wobei die Gradient-Norm durch die erste Ableitung einer Funktion E = f(x) mittels der quantenchemischen Methode erhalten wird, mit E=Gesamtenergie der optimierten Startgeometrie und x=Kernkoordinaten des Moleküls in der optimierten Startgeometrie,
   B4.1 wenn die Gradient-Norm B3 $\nabla$ $0 \le \nabla \le 0{,}07$ $E_h$ $a_0^{-1}$ beträgt, dann Einordnung der optimierten Startgeometrie als eine Vorstufe für den Übergangszustand der chemischen Reaktion und Fortführung des Verfahrens mit Schritt D1, oder
   B4.2 wenn die Gradient-Norm B3 $\nabla$ > $0{,}07$ $E_h$ $a_0^{-1}$ beträgt, dann Ermittlung der Vorstufe für den Übergangszustand der chemischen Reaktion ausgehend von der optimierten Startgeometrie durch ein Verfahren umfassend die folgenden Schritte:

   **C Ermittlung der Vorstufe für den Übergangszustand der chemischen Reaktion**
   C1 Variation der optimierten Startgeometrie unter Anwendung eines Monte-Carlo-Algorithmus wobei
   C1.1 wenigstens ein Atom aus dem in Schritt B1 ausgewählten Funktionsraum zufällig ausgewählt wird,
   C1.2 ein Vektor für eine Auslenkung des in Schritt C1.1 gewählten Atoms zufällig ausgewählt wird,
   C1.3 das in Schritt C1.1 ausgewählte Atom anhand des Vektors aus Schritt C1.2 aus seiner Position in der optimierten Startgeometrie ausgelenkt wird, so dass eine Vorstufe für den Übergangszustand der chemischen Reaktion erhalten wird,
   C2 Geometrieoptimierung der Vorstufe für den Übergangszustand mittels der quantenchemischen Methode und mit der Randbedingung, dass die mindestens eine Bindung aus Schritt A2 die Länge hat, die in der Schritt C1.3 ermittelt wurde,
   C3 Ermittlung der Gradient-Norm C3 für die Vorstufe für den Übergangszustand aus Schritt C2, wobei die Gradient-Norm durch die erste Ableitung der Funktion E = f(x) mittels der quantenchemischen Methode erhalten wird, mit E=Gesamtenergie der Vorstufe für den Übergangszustand und x=Kernkoordinaten des Moleküls in der Vorstufe für den Übergangszustand,
   C4.1 wenn die Gradient-Norm C3 $\nabla$ $0 \le \nabla \le 0{,}07$ $E_h$ $a_0^{-1}$ beträgt, dann Fortführung des Verfahrens mit Schritt D1, oder
   C4.2 wenn die Gradient-Norm C3 $\nabla$ > $0{,}07$ $E_h$ $a_0^{-1}$ beträgt, dann Wiederholung der Schritte C1 bis C3 bis eine Gradient-Norm C3 $0 \le \nabla \le 0{,}07$ $E_h$ $a_0^{-1}$ erhalten wird, wobei

      (a) für den Fall, dass die Schritte C1 bis C3 ein Mal durchgeführt wurden, in Schritt C1 die Geometrie der Vorstufe für den Übergangszustand variiert wird, wenn der Wert ihrer Gradient-Norm C3 niedriger ist als der Wert der Gradient-Norm B3 der optimierten Startgeometrie, oder
      (b) für den Fall dass die Schritte C1 bis C3 mehr als ein Mal durchgeführt wurden, in Schritt C1 die Geometrie der optimierten Startgeometrie oder derjenigen Vorstufe für den Übergangszustand aus den vorrangegangen Wiederholungen variiert wird, welche im Vergleich zu allen bisher erhaltenen Gradient-Normen C3 und B3 den niedrigsten Wert für die Gradient-Norm C3 oder B3 aufweist,

**D Ermittlung des Übergangszustandes**
D1 Relaxation der Vorstufe aus Schritt C4.1 oder der Vorstufe aus Schritt B4.1 mittels der quantenchemischen Methode und eines Pseudo-Newton-Raphson-Algorithmus, so dass der Übergangszustand erhalten wird,
D2 gegebenenfalls Ermittlung eines Gleichgewichtszustands durch Auslenkung des Übergangszustands, so dass ein ausgelenkter Übergangszustand erhalten wird und Relaxation des ausgelenkten Übergangszustands mittels der quantenchemischen Methode, so dass ein Gleichgewichtszustand erhalten wird.

2. Verfahren nach Anspruch 1, **dadurch gekennzeichnet, dass** die quantenchemische Methode aus Schritten **B2, B3, C2, C3, D1** und **D2** eine semiempirische Methode, Dichte-Funktional-theoretische Methode oder eine Annäherung der Schrödinger-Gleichung ist, insbesondere ist die quantenchemische Methode aus Schritten **B2, B3, C2, C3, D1** und **D2** eine Dichte-Funktional-theoretische Methode.

3. Verfahren nach Anspruch 1 oder 2, **dadurch gekennzeichnet, dass** die chemische Reaktion eine Synthese ist ausgewählt aus der Gruppe bestehend aus Polymersynthesen, insbesondere Polyurethansynthesen, Synthesen von Monomeren für Polymerisationsreaktionen, industriell benötigte Grundchemikalien, Additiven, Tensiden und pharmakologischen Wirkstoffen.

4. Verfahren nach einem der vorstehenden Ansprüche, **dadurch gekennzeichnet, dass** in Schritt **A1** mindestens zwei Moleküle I und II bereitgestellt werden und in Schritt **A2** alternativ oder zusätzlich zu der mindestens einen Bindung auch mindestens ein Abstand zwischen mindestens einem Atom aus Molekül I und mindestens einem Atom aus Molekül II sowie die Länge des mindestens einen Abstands ausgewählt werden können, wobei die Länge des Abstands insbesondere höchstens 230 pm beträgt.

5. Verfahren nach Anspruch 4, **dadurch gekennzeichnet, dass** Molekül I ein Katalysator für die chemische Reaktion ist, insbesondere für eine Polymersynthese, und Molekül II ein Edukt der chemischen Reaktion.

6. Verfahren nach Anspruch 4 oder 5, **dadurch gekennzeichnet, dass** Molekül I eine Größe von $\leq 100$ Atomen aufweist und Molekül II eine Größe von $\leq 100$ Atomen aufweist, bevorzugt soll die Summe der Atome aus Molekül I und aus Molekül II $\leq 100$ Atome betragen.

7. Verfahren gemäß einem der Ansprüche 1 bis 6, **dadurch gekennzeichnet, dass** Information über den gemäß Schritt D.1 ermittelten Übergangszustand und/oder der gemäß Schritt D.2 ermittelten Gleichgewichtszustand einem Benutzer mitgeteilt wird.

8. Verfahren gemäß einem der Ansprüche 1 bis 6, **dadurch gekennzeichnet, dass** Information über den gemäß Schritt D.1 ermittelten Übergangszustand und/oder der gemäß Schritt D.2 ermittelten Gleichgewichtszustand durch einen Benutzer empfangen wird.

9. Verfahren gemäß Anspruch 5 oder 6, **dadurch gekennzeichnet, dass** nach Schritt D.1 und/oder nach Schritt D.2 das Molekül I synthetisiert wird.

10. Verfahren gemäß Anspruch 5 oder 6, **dadurch gekennzeichnet, dass** nach Schritt D.1 und/oder nach Schritt D.2 unter eine chemische Reaktion mit dem Molekül I als Katalysator durchgeführt wird.

11. Verfahren gemäß Anspruch 5, 6 oder 10, **dadurch gekennzeichnet, dass** nach Schritt D.1 und/oder nach Schritt D.2 unter eine chemische Reaktion mit dem Molekül II als Reaktionspartner durchgeführt wird.

12. System zur Datenverarbeitung umfassend Mittel zur Ausführung eines Verfahrens umfassend die Schritte:

**A Erzeugen einer Startgeometrie**
A1 Bereitstellung der dreidimensionalen Darstellung mindestens eines Moleküls im energetischen Grundzustand,
A2 Auswahl von mindestens einer Bindung des mindestens einen Moleküls und Auswahl einer Länge der Bindung, wobei die ausgewählte Länge nicht der Länge der Bindung im energetischen Grundzustand des Moleküls entspricht, so dass eine Startgeometrie für die chemische Reaktion erhalten wird,

A3 Dreidimensionale Darstellung der Startgeometrie in kartesischen und/oder internen Koordinaten,

**B Ermittlung einer optimierten Startgeometrie**

B1 Festlegung eines Funktionsraumes welcher die mindestens eine Bindung aus Schritt A2 und die durch diese Bindung verbundenen Atome umfasst,

B2 Geometrieoptimierung der Startgeometrie unter Zugrundelegung des in Schritt B1 ausgewählten Funktionsraums mittels einer quantenchemischen Methode und mit der Randbedingung, dass die in Schritt A2 ausgewählte Länge der mindestens einen Bindung konstant gehalten wird, so dass eine optimierte Startgeometrie erhalten wird,

B3 Ermittlung der Gradient-Norm B3 für die optimierte Startgeometrie, wobei die Gradient-Norm durch die erste Ableitung einer Funktion E = f(x) mittels der quantenchemischen Methode erhalten wird, mit E=Gesamtenergie der optimierten Startgeometrie und x=Kernkoordinaten des Moleküls in der optimierten Startgeometrie,

B4.1 wenn die Gradient-Norm B3 $\nabla$ $0 \leq \nabla \leq 0{,}07$ $E_h$ $a_0^{-1}$ beträgt, dann Einordnung der optimierten Startgeometrie als eine Vorstufe für den Übergangszustand der chemischen Reaktion und Fortführung des Verfahrens mit Schritt D1, oder

B4.2 wenn die Gradient-Norm B3 $\nabla > 0{,}07$ $E_h$ $a_0^{-1}$ beträgt, dann Ermittlung der Vorstufe für den Übergangszustand der chemischen Reaktion ausgehend von der optimierten Startgeometrie durch ein Verfahren umfassend die folgenden Schritte:

**C Ermittlung der Vorstufe für den Übergangszustand der chemischen Reaktion**

C1 Variation der optimierten Startgeometrie unter Anwendung eines Monte-Carlo-Algorithmus wobei

C1.1 wenigstens ein Atom aus dem in Schritt B1 ausgewählten Funktionsraum zufällig ausgewählt wird,

C1.2 ein Vektor für eine Auslenkung des in Schritt C1.1 gewählten Atoms zufällig ausgewählt wird,

C1.3 das in Schritt C1.1 ausgewählte Atom anhand des Vektors aus Schritt C1.2 aus seiner Position in der optimierten Startgeometrie ausgelenkt wird, so dass eine Vorstufe für den Übergangszustand der chemischen Reaktion erhalten wird,

C2 Geometrieoptimierung der Vorstufe für den Übergangszustand mittels der quantenchemischen Methode und mit der Randbedingung, dass die mindestens eine Bindung aus Schritt A2 die Länge hat, die in der Schritt C1.3 ermittelt wurde,

C3 Ermittlung der Gradient-Norm C3 für die Vorstufe für den Übergangszustand aus Schritt C2, wobei die Gradient-Norm durch die erste Ableitung der Funktion E = f(x) mittels der quantenchemischen Methode erhalten wird, mit E=Gesamtenergie der Vorstufe für den Übergangszustand und x=Kernkoordinaten des Moleküls in der Vorstufe für den Übergangszustand,

C4.1 wenn die Gradient-Norm C3 $\nabla$ $0 \leq \nabla \leq 0{,}07$ $E_h$ $a_0^{-1}$ beträgt, dann Fortführung des Verfahrens mit Schritt D1, oder

C4.2 wenn die Gradient-Norm C3 $\nabla > 0{,}07$ $E_h$ $a_0^{-1}$ beträgt, dann Wiederholung der Schritte C1 bis C3 bis eine Gradient-Norm C3 $0 \leq \nabla \leq 0{,}07$ $E_h$ $a_0^{-1}$ erhalten wird, wobei

(a) für den Fall, dass die Schritte C1 bis C3 ein Mal durchgeführt wurden, in Schritt C1 die Geometrie der Vorstufe für den Übergangszustand variiert wird, wenn der Wert ihrer Gradient-Norm C3 niedriger ist als der Wert der Gradient-Norm B3 der optimierten Startgeometrie, oder

(b) für den Fall dass die Schritte C1 bis C3 mehr als ein Mal durchgeführt wurden, in Schritt C1 die Geometrie der optimierten Startgeometrie oder derjenigen Vorstufe für den Übergangszustand aus den vorrangegangenen Wiederholungen variiert wird, welche im Vergleich zu allen bisher erhaltenen Gradient-Normen C3 und B3 den niedrigsten Wert für die Gradient-Norm C3 oder B3 aufweist,

**D Ermittlung des Übergangszustandes**

D1 Relaxation der Vorstufe aus Schritt C4.1 oder der Vorstufe aus Schritt B4.1 mittels der quantenchemischen Methode und eines Pseudo-Newton-Raphson-Algorithmus, so dass der Übergangszustand erhalten wird,

D2 gegebenenfalls Ermittlung eines Gleichgewichtszustands durch Auslenkung des Übergangszustands, so dass ein ausgelenkter Übergangszustand erhalten wird und Relaxation des ausgelenkten Übergangszustands mittels der quantenchemischen Methode, so dass ein Gleichgewichtszustand erhalten wird.

13. Computerprogramm, umfassend Befehle, die bei der Ausführung des Programms durch einen Computer diesen veranlassen, die folgenden Schritte eines Verfahrens durchzuführen:

**A Erzeugen einer Startgeometrie**

A1 Bereitstellung der dreidimensionalen Darstellung mindestens eines Moleküls im energetischen Grundzustand,

A2 Auswahl von mindestens einer Bindung des mindestens einen Moleküls und Auswahl einer Länge der Bindung, wobei die ausgewählte Länge nicht der Länge der Bindung im energetischen Grundzustand des Moleküls entspricht, so dass eine Startgeometrie für die chemische Reaktion erhalten wird,

A3 Dreidimensionale Darstellung der Startgeometrie in kartesischen und/oder internen Koordinaten,

**B Ermittlung einer optimierten Startgeometrie**

B1 Festlegung eines Funktionsraumes welcher die mindestens eine Bindung aus Schritt A2 und die durch diese Bindung verbundenen Atome umfasst,

B2 Geometrieoptimierung der Startgeometrie unter Zugrundelegung des in Schritt B1 ausgewählten Funktionsraums mittels einer quantenchemischen Methode und mit der Randbedingung, dass die in Schritt A2 ausgewählte Länge der mindestens einen Bindung konstant gehalten wird, so dass eine optimierte Startgeometrie erhalten wird,

B3 Ermittlung der Gradient-Norm B3 für die optimierte Startgeometrie, wobei die Gradient-Norm durch die erste Ableitung einer Funktion E = f(x) mittels der quantenchemischen Methode erhalten wird, mit E=Gesamtenergie der optimierten Startgeometrie und x=Kernkoordinaten des Moleküls in der optimierten Startgeometrie,

B4.1 wenn die Gradient-Norm B3 $\nabla$ $0 \leq \nabla \leq 0{,}07$ $E_h\,a_0^{-1}$ beträgt, dann Einordnung der optimierten Startgeometrie als eine Vorstufe für den Übergangszustand der chemischen Reaktion und Fortführung des Verfahrens mit Schritt D1, oder

B4.2 wenn die Gradient-Norm B3 $\nabla > 0{,}07$ $E_h\,a_0^{-1}$ beträgt, dann Ermittlung der Vorstufe für den Übergangszustand der chemischen Reaktion ausgehend von der optimierten Startgeometrie durch ein Verfahren umfassend die folgenden Schritte:

**C Ermittlung der Vorstufe für den Übergangszustand der chemischen Reaktion**

C1 Variation der optimierten Startgeometrie unter Anwendung eines Monte-Carlo-Algorithmus wobei

C1.1 wenigstens ein Atom aus dem in Schritt B1 ausgewählten Funktionsraum zufällig ausgewählt wird,

C1.2 ein Vektor für eine Auslenkung des in Schritt C1.1 gewählten Atoms zufällig ausgewählt wird,

C1.3 das in Schritt C1.1 ausgewählte Atom anhand des Vektors aus Schritt C1.2 aus seiner Position in der optimierten Startgeometrie ausgelenkt wird, so dass eine Vorstufe für den Übergangszustand der chemischen Reaktion erhalten wird,

C2 Geometrieoptimierung der Vorstufe für den Übergangszustand mittels der quantenchemischen Methode und mit der Randbedingung, dass die mindestens eine Bindung aus Schritt A2 die Länge hat, die in der Schritt C1.3 ermittelt wurde,

C3 Ermittlung der Gradient-Norm C3 für die Vorstufe für den Übergangszustand aus Schritt C2, wobei die Gradient-Norm durch die erste Ableitung der Funktion E = f(x) mittels der quantenchemischen Methode erhalten wird, mit E=Gesamtenergie der Vorstufe für den Übergangszustand und x=Kernkoordinaten des Moleküls in der Vorstufe für den Übergangszustand,

C4.1 wenn die Gradient-Norm C3 $\nabla$ $0 \leq \nabla \leq 0{,}07$ $E_h\,a_0^{-1}$ beträgt, dann Fortführung des Verfahrens mit Schritt D1, oder

C4.2 wenn die Gradient-Norm C3 $\nabla > 0{,}07$ $E_h\,a_0^{-1}$ beträgt, dann Wiederholung der Schritte C1 bis C3 bis eine Gradient-Norm C3 $0 \leq \nabla \leq 0{,}07$ $E_h\,a_0^{-1}$ erhalten wird, wobei

(a) für den Fall, dass die Schritte C1 bis C3 ein Mal durchgeführt wurden, in Schritt C1 die Geometrie der Vorstufe für den Übergangszustand variiert wird, wenn der Wert ihrer Gradient-Norm C3 niedriger ist als der Wert der Gradient-Norm B3 der optimierten Startgeometrie, oder

(b) für den Fall dass die Schritte C1 bis C3 mehr als ein Mal durchgeführt wurden, in Schritt C1 die Geometrie der optimierten Startgeometrie oder derjenigen Vorstufe für den Übergangszustand aus den vorrangegangenen Wiederholungen variiert wird, welche im Vergleich zu allen bisher erhaltenen Gradient-Normen C3 und B3 den niedrigsten Wert für die Gradient-Norm C3 oder B3 aufweist,

**D Ermittlung des Übergangszustandes**

D1 Relaxation der Vorstufe aus Schritt C4.1 oder der Vorstufe aus Schritt B4.1 mittels der quantenchemischen Methode und eines Pseudo-Newton-Raphson-Algorithmus, so dass der Übergangszustand erhalten wird,

D2 gegebenenfalls Ermittlung eines Gleichgewichtszustands durch Auslenkung des Übergangszustands, so dass ein ausgelenkter Übergangszustand erhalten wird und Relaxation des ausgelenkten Übergangszustands mittels der quantenchemischen Methode, so dass ein Gleichgewichtszustand erhalten wird.

**14.** Computerlesbares Speichermedium, umfassend Befehle, die bei der Ausführung durch einen Computer diesen veranlassen, die folgenden Schritte eines Verfahrens durchzuführen:

**A Erzeugen einer Startgeometrie**

A1 Bereitstellung der dreidimensionalen Darstellung mindestens eines Moleküls im energetischen Grundzustand,

A2 Auswahl von mindestens einer Bindung des mindestens einen Moleküls und Auswahl einer Länge der Bindung, wobei die ausgewählte Länge nicht der Länge der Bindung im energetischen Grundzustand des Moleküls entspricht, so dass eine Startgeometrie für die chemische Reaktion erhalten wird,

A3 Dreidimensionale Darstellung der Startgeometrie in kartesischen und/oder internen Koordinaten,

**B Ermittlung einer optimierten Startgeometrie**

B1 Festlegung eines Funktionsraumes welcher die mindestens eine Bindung aus Schritt A2 und die durch diese Bindung verbundenen Atome umfasst,

B2 Geometrieoptimierung der Startgeometrie unter Zugrundelegung des in Schritt B1 ausgewählten Funktionsraums mittels einer quantenchemischen Methode und mit der Randbedingung, dass die in Schritt A2 ausgewählte Länge der mindestens einen Bindung konstant gehalten wird, so dass eine optimierte Startgeometrie erhalten wird,

B3 Ermittlung der Gradient-Norm B3 für die optimierte Startgeometrie, wobei die Gradient-Norm durch die erste Ableitung einer Funktion E = f(x) mittels der quantenchemischen Methode erhalten wird, mit E=Gesamtenergie der optimierten Startgeometrie und x=Kernkoordinaten des Moleküls in der optimierten Startgeometrie,

B4.1 wenn die Gradient-Norm B3 $\nabla$ $0 \leq \nabla \leq 0{,}07$ $E_h$ $a_0^{-1}$ beträgt, dann Einordnung der optimierten Startgeometrie als eine Vorstufe für den Übergangszustand der chemischen Reaktion und Fortführung des Verfahrens mit Schritt D1, oder

B4.2 wenn die Gradient-Norm B3 $\nabla > 0{,}07$ $E_h$ $a_0^{-1}$ beträgt, dann Ermittlung der Vorstufe für den Übergangszustand der chemischen Reaktion ausgehend von der optimierten Startgeometrie durch ein Verfahren umfassend die folgenden Schritte:

**C Ermittlung der Vorstufe für den Übergangszustand der chemischen Reaktion**

C1 Variation der optimierten Startgeometrie unter Anwendung eines Monte-Carlo-Algorithmus wobei

C1.1 wenigstens ein Atom aus dem in Schritt B1 ausgewählten Funktionsraum zufällig ausgewählt wird,

C1.2 ein Vektor für eine Auslenkung des in Schritt C1.1 gewählten Atoms zufällig ausgewählt wird,

C1.3 das in Schritt C1.1 ausgewählte Atom anhand des Vektors aus Schritt C1.2 aus seiner Position in der optimierten Startgeometrie ausgelenkt wird, so dass eine Vorstufe für den Übergangszustand der chemischen Reaktion erhalten wird,

C2 Geometrieoptimierung der Vorstufe für den Übergangszustand mittels der quantenchemischen Methode und mit der Randbedingung, dass die mindestens eine Bindung aus Schritt A2 die Länge hat, die in der Schritt C1.3 ermittelt wurde,

C3 Ermittlung der Gradient-Norm C3 für die Vorstufe für den Übergangszustand aus Schritt C2, wobei die Gradient-Norm durch die erste Ableitung der Funktion E = f(x) mittels der quantenchemischen Methode erhalten wird, mit E=Gesamtenergie der Vorstufe für den Übergangszustand und x=Kernkoordinaten des Moleküls in der Vorstufe für den Übergangszustand,

C4.1 wenn die Gradient-Norm C3 $\nabla$ $0 \leq \nabla \leq 0{,}07$ $E_h$ $a_0^{-1}$ beträgt, dann Fortführung des Verfahrens mit Schritt D1, oder

C4.2 wenn die Gradient-Norm C3 $\nabla > 0{,}07$ $E_h$ $a_0^{-1}$ beträgt, dann Wiederholung der Schritte C1 bis C3 bis eine Gradient-Norm C3 $0 \leq \nabla \leq 0{,}07$ $E_h$ $a_0^{-1}$ erhalten wird, wobei

(a) für den Fall, dass die Schritte C1 bis C3 ein Mal durchgeführt wurden, in Schritt C1 die Geometrie der Vorstufe für den Übergangszustand variiert wird, wenn der Wert ihrer Gradient-Norm C3 niedriger ist als der Wert der Gradient-Norm B3 der optimierten Startgeometrie, oder

(b) für den Fall dass die Schritte C1 bis C3 mehr als ein Mal durchgeführt wurden, in Schritt C1 die Geometrie der optimierten Startgeometrie oder derjenigen Vorstufe für den Übergangszustand aus den vorrangegangen Wiederholungen variiert wird, welche im Vergleich zu allen bisher erhaltenen Gradient-Normen C3 und B3 den niedrigsten Wert für die Gradient-Norm C3 oder B3 aufweist,

**D Ermittlung des Übergangszustandes**

D1 Relaxation der Vorstufe aus Schritt C4.1 oder der Vorstufe aus Schritt B4.1 mittels der quantenchemischen Methode und eines Pseudo-Newton-Raphson-Algorithmus, so dass der Übergangszustand erhalten wird,

D2 gegebenenfalls Ermittlung eines Gleichgewichtszustands durch Auslenkung des Übergangszustands, so dass ein ausgelenkter Übergangszustand erhalten wird und Relaxation des ausgelenkten Übergangszustands mittels der quantenchemischen Methode, so dass ein Gleichgewichtszustand erhalten wird.

15. Verwendung eines Computerprogramms nach Anspruch 13 oder eines computerlesbaren Speichermediums nach Anspruch 14 zur Bewertung von Übergangszuständen einer chemischen Reaktion, insbesondere einer Polymersynthese.

**Claims**

1. Computer-implemented method of calculating transition states of a chemical reaction, comprising the steps of

**A Generating a starting geometry**
A1 providing the three-dimensional representation of at least one molecule at ground state energy,
A2 selecting at least one bond of the at least one molecule and selecting a length of the bond, where the selected length does not correspond to the length of the bond at ground state energy of the molecule, such that a starting geometry for the chemical reaction is obtained,
A3 representing the starting geometry in three dimensions in Cartesian and/or internal coordinates,

**B Ascertaining an optimized starting geometry**
B1 defining a function space encompassing the at least one bond from step A2 and the atoms joined by this bond,
B2 optimizing the geometry of the starting geometry on the basis of the function space selected in step B1 by means of a quantum-chemical method and with the boundary condition that the length of the at least one bond selected in step A2 is kept constant, such that an optimized starting geometry is obtained,
B3 ascertaining the gradient norm B3 for the optimized starting geometry, where the gradient norm is obtained via the first derivative of a function $E = f(x)$ by means of the quantum-chemical method, with E = total energy of the optimized starting geometry and x = nuclear coordinates of the molecule in the optimized starting geometry,
B4.1 when the gradient norm B3 $\nabla$ is $0 \leq \nabla \leq 0.07\ E_h\ a_0^{-1}$, classifying the optimized starting geometry as a precursor to the transition state of the chemical reaction and continuing the method with step D1, or
B4.2 when the gradient norm B3 V is $> 0.07\ E_h\ a_0^{-1}$, ascertaining the precursor to the transition state of the chemical reaction proceeding from the optimized starting geometry by a method comprising the following steps:

**C Ascertaining the precursor to the transition state of the chemical reaction**
C1 varying the optimized starting geometry using a Monte Carlo algorithm, wherein
C1.1 at least one atom from the function space selected in step B1 is selected at random,
C1.2 a vector for a deflection of the atom chosen in step C1.1 is selected at random,
C1.3 the atom selected in step C1.1 is deflected from its position in the optimized starting geometry using the vector from step C1.2, so as to obtain a precursor to the transition state of the chemical reaction,
C2 optimizing the geometry of the precursor to the transition state by means of the quantum-chemical method and with the boundary condition that the at least one bond from step A2 has the length that was ascertained in step C1.3,
C3 ascertaining the gradient norm C3 for the precursor to the transition state from step C2, where the gradient norm is obtained via the first derivative of the function $E = f(x)$ by means of the quantum-chemical method, with E = total energy of the precursor to the transition state and x = nuclear coordinates of the molecule in the precursor to the transition state,
C4.1 when the gradient norm C3 V is $0 \leq \nabla \leq 0.07\ E_h\ a_0^{-1}$, continuing the method with step D1, or
C4.2 when the gradient norm C3 V is $> 0.07\ E_h\ a_0^{-1}$, repeating steps C1 to C3 until a gradient norm C3 of $0 \leq \nabla \leq 0.07\ E_h\ a_0^{-1}$ is obtained, wherein

(a) if steps C1 to C3 have been performed once, the geometry of the precursor to the transition state is varied in step C1 when the value of its gradient norm C3 is lower than the value of the gradient norm B3 of the optimized starting geometry, or
(b) if steps C1 to C3 have been performed more than once, the geometry of the optimized starting geometry or that precursor to the transition state from the preceding repetitions that has the lowest value for the gradient norm C3 or B3 compared to all the gradient norms C3 and B3 previously obtained is varied in step C1,

**D Ascertaining the transition state**

D1 relaxing the precursor from step C4.1 or the precursor from step B4.1 by means of the quantum-chemical method and a pseudo-Newton-Raphson algorithm, such that the transition state is obtained,
D2 optionally ascertaining an equilibrium state by deflecting the transition state, such that a deflected transition state is obtained, and relaxing the deflected transition state by means of the quantum-chemical method, such that an equilibrium state is obtained.

2. Method according to Claim 1, **characterized in that** the quantum-chemical method from steps **B2, B3, C2, C3, D1** and **D2** is a semiempirical method, density functional theory method or an approximation of the Schrödinger equation, the quantum-chemical method from steps **B2, B3, C2, C3, D1** and **D2** especially being a density functional theory method.

3. Method according to Claim 1 or 2, **characterized in that** the chemical reaction is a synthesis selected from the group consisting of polymer syntheses, especially polyurethane syntheses, syntheses of monomers for polymerization reactions, industrially required commodity chemicals, additives, surfactants and active pharmacological ingredients.

4. Method according to any of the preceding claims, **characterized in that**, in step **A1,** at least two molecules I and II are provided and, in step **A2,** alternatively or additionally to the at least one bond, at least one distance between at least one atom from molecule I and at least one atom from molecule II and the length of the at least one distance may also be selected, where the length of the distance is especially not more than 230 pm.

5. Method according to Claim 4, **characterized in that** molecule I is a catalyst for the chemical reaction, especially for a polymer synthesis, and molecule II is a reactant in the chemical reaction.

6. Method according to Claim 4 or 5, **characterized in that** molecule I has a size of $\leq 100$ atoms and molecule II has a size of $\leq 100$ atoms, and the sum total of the atoms from molecule I and from molecule II should preferably be $\leq 100$ atoms.

7. Method according to any of Claims 1 to 6, **characterized in that** information about the transition state ascertained according to step D.1 and/or the equilibrium state ascertained according to step D.2 is communicated to a user.

8. Method according to any of Claims 1 to 6, **characterized in that** information about the transition state ascertained according to step D.1 and/or the equilibrium state ascertained according to step D.2 is received by a user.

9. Method according to Claim 5 or 6, **characterized in that** the molecule I is synthesized after step D.1 and/or after step D.2.

10. Method according to Claim 5 or 6, **characterized in that** a chemical reaction is performed after step D.1 and/or after step D.2 with the molecule I as catalyst.

11. Method according to Claim 5, 6 or 10, **characterized in that** a chemical reaction is performed after step D.1 and/or after step D.2 with the molecule II as co-reactant.

12. System for data processing, comprising means of executing a method comprising the steps of:

**A Generating a starting geometry**
A1 providing the three-dimensional representation of at least one molecule at ground state energy,
A2 selecting at least one bond of the at least one molecule and selecting a length of the bond, where the selected length does not correspond to the length of the bond at ground state energy of the molecule, such that a starting geometry for the chemical reaction is obtained,
A3 representing the starting geometry in three dimensions in Cartesian and/or internal coordinates,
**B Ascertaining an optimized starting geometry**
B1 defining a function space encompassing the at least one bond from step A2 and the atoms joined by this bond,
B2 optimizing the geometry of the starting geometry on the basis of the function space selected in step B1 by means of a quantum-chemical method and with the boundary condition that the length of the at least one bond selected in step A2 is kept constant, such that an optimized starting geometry is obtained,
B3 ascertaining the gradient norm B3 for the optimized starting geometry, where the gradient norm is obtained via the first derivative of a function E = f(x) by means of the quantum-chemical method, with E = total energy of the optimized starting geometry and x = nuclear coordinates of the molecule in the optimized starting geometry,
B4.1 when the gradient norm B3 V is $0 \leq \nabla \leq 0.07\ E_h\ a_0^{-1}$, classifying the optimized starting geometry as a precursor to the transition state of the chemical reaction and continuing the method with step D1, or

B4.2 when the gradient norm B3 V is > 0.07 $E_h$ $a_0^{-1}$, ascertaining the precursor to the transition state of the chemical reaction proceeding from the optimized starting geometry by a method comprising the following steps:

**C Ascertaining the precursor to the transition state of the chemical reaction**

C1 varying the optimized starting geometry using a Monte Carlo algorithm, wherein

C1.1 at least one atom from the function space selected in step B1 is selected at random,

C1.2 a vector for a deflection of the atom chosen in step C1.1 is selected at random,

C1.3 the atom selected in step C1.1 is deflected from its position in the optimized starting geometry using the vector from step C1.2, so as to obtain a precursor to the transition state of the chemical reaction,

C2 optimizing the geometry of the precursor to the transition state by means of the quantum-chemical method and with the boundary condition that the at least one bond from step A2 has the length that was ascertained in step C1.3,

C3 ascertaining the gradient norm C3 for the precursor to the transition state from step C2, where the gradient norm is obtained via the first derivative of the function E = f(x) by means of the quantum-chemical method, with E = total energy of the precursor to the transition state and x = nuclear coordinates of the molecule in the precursor to the transition state,

C4.1 when the gradient norm C3 V is $0 \leq \nabla \leq 0.07$ $E_h$ $a_0^{-1}$, continuing the method with step D1, or

C4.2 when the gradient norm C3 V is > 0.07 $E_h$ $a_0^{-1}$, repeating steps C1 to C3 until a gradient norm C3 of $0 \leq V \leq 0.07$ $E_h$ $a_0^{-1}$ is obtained, wherein

(a) if steps C1 to C3 have been performed once, the geometry of the precursor to the transition state is varied in step C1 when the value of its gradient norm C3 is lower than the value of the gradient norm B3 of the optimized starting geometry, or

(b) if steps C1 to C3 have been performed more than once, the geometry of the optimized starting geometry or that precursor to the transition state from the preceding repetitions that has the lowest value for the gradient norm C3 or B3 compared to all the gradient norms C3 and B3 previously obtained is varied in step C1,

**D Ascertaining the transition state**

D1 relaxing the precursor from step C4.1 or the precursor from step B4.1 by means of the quantum-chemical method and a pseudo-Newton-Raphson algorithm, such that the transition state is obtained,

D2 optionally ascertaining an equilibrium state by deflecting the transition state, such that a deflected transition state is obtained, and relaxing the deflected transition state by means of the quantum-chemical method, such that an equilibrium state is obtained.

**13.** Computer program comprising commands that, on execution of the program by a computer, cause it to perform the following steps of a method:

**A Generating a starting geometry**

A1 providing the three-dimensional representation of at least one molecule at ground state energy,

A2 selecting at least one bond of the at least one molecule and selecting a length of the bond, where the selected length does not correspond to the length of the bond at ground state energy of the molecule, such that a starting geometry for the chemical reaction is obtained,

A3 representing the starting geometry in three dimensions in Cartesian and/or internal coordinates,

**B Ascertaining an optimized starting geometry**

B1 defining a function space encompassing the at least one bond from step A2 and the atoms joined by this bond,

B2 optimizing the geometry of the starting geometry on the basis of the function space selected in step B1 by means of a quantum-chemical method and with the boundary condition that the length of the at least one bond selected in step A2 is kept constant, such that an optimized starting geometry is obtained,

B3 ascertaining the gradient norm B3 for the optimized starting geometry, where the gradient norm is obtained via the first derivative of a function E = f(x) by means of the quantum-chemical method, with E = total energy of the optimized starting geometry and x = nuclear coordinates of the molecule in the optimized starting geometry,

B4.1 when the gradient norm B3 V is $0 \leq \nabla \leq 0.07$ $E_h$ $a_0^{-1}$, classifying the optimized starting geometry as a precursor to the transition state of the chemical reaction and continuing the method with step D1, or

B4.2 when the gradient norm B3 V is > 0.07 $E_h$ $a_0^{-1}$, ascertaining the precursor to the transition state of the chemical reaction proceeding from the optimized starting geometry by a method comprising the following steps:

**C Ascertaining the precursor to the transition state of the chemical reaction**

C1 varying the optimized starting geometry using a Monte Carlo algorithm, wherein

C1.1 at least one atom from the function space selected in step B1 is selected at random,

C1.2 a vector for a deflection of the atom chosen in step C1.1 is selected at random,

C1.3 the atom selected in step C1.1 is deflected from its position in the optimized starting geometry using the vector from step C1.2, so as to obtain a precursor to the transition state of the chemical reaction,

C2 optimizing the geometry of the precursor to the transition state by means of the quantum-chemical method and with the boundary condition that the at least one bond from step A2 has the length that was ascertained in step C1.3,

C3 ascertaining the gradient norm C3 for the precursor to the transition state from step C2, where the gradient norm is obtained via the first derivative of the function $E = f(x)$ by means of the quantum-chemical method, with $E$ = total energy of the precursor to the transition state and $x$ = nuclear coordinates of the molecule in the precursor to the transition state,

C4.1 when the gradient norm C3 V is $0 \leq \nabla \leq 0.07$ $E_h$ $a_0^{-1}$, continuing the method with step D1, or

C4.2 when the gradient norm C3 V is > 0.07 $E_h$ $a_0^{-1}$, repeating steps C1 to C3 until a gradient norm C3 of $0 \leq \nabla \leq 0.07$ $E_h$ $a_0^{-1}$ is obtained, wherein

(a) if steps C1 to C3 have been performed once, the geometry of the precursor to the transition state is varied in step C1 when the value of its gradient norm C3 is lower than the value of the gradient norm B3 of the optimized starting geometry, or

(b) if steps C1 to C3 have been performed more than once, the geometry of the optimized starting geometry or that precursor to the transition state from the preceding repetitions that has the lowest value for the gradient norm C3 or B3 compared to all the gradient norms C3 and B3 previously obtained is varied in step C1,

**D Ascertaining the transition state**

D1 relaxing the precursor from step C4.1 or the precursor from step B4.1 by means of the quantum-chemical method and a pseudo-Newton-Raphson algorithm, such that the transition state is obtained,

D2 optionally ascertaining an equilibrium state by deflecting the transition state, such that a deflected transition state is obtained, and relaxing the deflected transition state by means of the quantum-chemical method, such that an equilibrium state is obtained.

14. Computer-readable storage medium comprising commands that, on execution by a computer, cause it to perform the following steps of a method:

**A Generating a starting geometry**

A1 providing the three-dimensional representation of at least one molecule at ground state energy,

A2 selecting at least one bond of the at least one molecule and selecting a length of the bond, where the selected length does not correspond to the length of the bond at ground state energy of the molecule, such that a starting geometry for the chemical reaction is obtained,

A3 representing the starting geometry in three dimensions in Cartesian and/or internal coordinates,

**B Ascertaining an optimized starting geometry**

B1 defining a function space encompassing the at least one bond from step A2 and the atoms joined by this bond,

B2 optimizing the geometry of the starting geometry on the basis of the function space selected in step B1 by means of a quantum-chemical method and with the boundary condition that the length of the at least one bond selected in step A2 is kept constant, such that an optimized starting geometry is obtained,

B3 ascertaining the gradient norm B3 for the optimized starting geometry, where the gradient norm is obtained via the first derivative of a function $E = f(x)$ by means of the quantum-chemical method, with $E$ = total energy of the optimized starting geometry and $x$ = nuclear coordinates of the molecule in the optimized starting geometry,

B4.1 when the gradient norm B3 V is $0 \leq \nabla \leq 0.07$ $E_h$ $a_0^{-1}$, classifying the optimized starting geometry as a precursor to the transition state of the chemical reaction and continuing the method with step D1, or

B4.2 when the gradient norm B3 V is > 0.07 $E_h$ $a_0^{-1}$, ascertaining the precursor to the transition state of the chemical reaction proceeding from the optimized starting geometry by a method comprising the following steps:

**C Ascertaining the precursor to the transition state of the chemical reaction**

C1 varying the optimized starting geometry using a Monte Carlo algorithm, wherein

C1.1 at least one atom from the function space selected in step B1 is selected at random,

C1.2 a vector for a deflection of the atom chosen in step C1.1 is selected at random,

C1.3 the atom selected in step C1.1 is deflected from its position in the optimized starting geometry using the vector from step C1.2, so as to obtain a precursor to the transition state of the chemical reaction,

C2 optimizing the geometry of the precursor to the transition state by means of the quantum-chemical method and with the boundary condition that the at least one bond from step A2 has the length that was ascertained in step C1.3,

C3 ascertaining the gradient norm C3 for the precursor to the transition state from step C2, where the gradient norm is obtained via the first derivative of the function $E = f(x)$ by means of the quantum-chemical method, with E = total energy of the precursor to the transition state and x = nuclear coordinates of the molecule in the precursor to the transition state,

C4.1 when the gradient norm C3 $\nabla$ is $0 \leq \leq 0.07$ $E_h$ $a_0^{-1}$, continuing the method with step D1, or

C4.2 when the gradient norm C3 V is > 0.07 $E_h$ $a_0^{-1}$, repeating steps C1 to C3 until a gradient norm C3 of $0 \leq \nabla \leq 0.07$ $E_h$ $a_0^{-1}$ is obtained, wherein

(a) if steps C1 to C3 have been performed once, the geometry of the precursor to the transition state is varied in step C1 when the value of its gradient norm C3 is lower than the value of the gradient norm B3 of the optimized starting geometry, or

(b) if steps C1 to C3 have been performed more than once, the geometry of the optimized starting geometry or that precursor to the transition state from the preceding repetitions that has the lowest value for the gradient norm C3 or B3 compared to all the gradient norms C3 and B3 previously obtained is varied in step C1,

**D Ascertaining the transition state**

D1 relaxing the precursor from step C4.1 or the precursor from step B4.1 by means of the quantum-chemical method and a pseudo-Newton-Raphson algorithm, such that the transition state is obtained,

D2 optionally ascertaining an equilibrium state by deflecting the transition state, such that a deflected transition state is obtained, and relaxing the deflected transition state by means of the quantum-chemical method, such that an equilibrium state is obtained.

15. Use of a computer program according to Claim 13 or of a computer-readable storage medium according to Claim 14 for evaluating transition states of a chemical reaction, especially a polymer synthesis.

**Revendications**

1. Procédé mis en œuvre par un ordinateur pour le calcul d'états de transition d'une réaction chimique, comprenant les étapes

A génération d'une géométrie de départ

A1 mise à disposition de la représentation tridimensionnelle d'au moins une molécule dans l'état énergétique de base,

A2 sélection d'au moins une liaison de ladite au moins une molécule et sélection d'une longueur de la liaison, la longueur sélectionnée ne correspondant pas à la longueur de la liaison dans l'état énergétique de base de la molécule, de telle sorte qu'une géométrie de départ pour la réaction chimique est obtenue,

A3 représentation tridimensionnelle de la géométrie de départ en coordonnées cartésiennes et/ou internes,

B détermination d'une géométrie de départ optimisée B1 définition d'un espace fonctionnel qui comprend ladite au moins une liaison de l'étape A2 et les atomes reliés par cette liaison,

B2 optimisation géométrique de la géométrique de départ sur la base de l'espace fonctionnel sélectionné dans l'étape B1 au moyen d'une méthode de chimie quantique et sous la condition limite selon laquelle la longueur sélectionnée dans l'étape A2 de ladite au moins une liaison est maintenue constante, de telle sorte qu'une géométrie de départ optimisée est obtenue,

B3 détermination de la norme de gradient B3 pour la géométrie de départ optimisée, la norme de gradient étant obtenue par la première dérivée d'une fonction $E = f(x)$ au moyen de la méthode de chimie quantique, avec E = énergie totale de la géométrie de départ optimisée et x = les coordonnées de noyau de la molécule dans la géométrie de départ optimisée,

B4.1 lorsque la norme de gradient B3 V répond à $0 \leq \nabla \leq 0,07$ $E_h$ $a_0^{-1}$, alors classification de la géométrie de départ optimisée comme un stade préliminaire à l'état de transition de la réaction chimique et poursuite du procédé par l'étape D1 ou

B4.2 lorsque la norme de gradient B3 V répond à > 0,07 $E_h$ $a_0^{-1}$, alors détermination du stade préliminaire à l'état de transition de la réaction chimique partant de la géométrie de départ optimisée par un procédé comprenant les étapes suivantes :

C détermination du stade préliminaire à l'état de transition de la réaction chimique

C1 variation de la géométrie de départ optimisée à l'aide d'un algorithme de Monte-Carlo

C1.1 au moins un atome de l'espace fonctionnel sélectionné dans l'étape B1 étant sélectionné aléatoirement,

C1.2 un vecteur pour une déviation de l'atome sélectionné dans l'étape C1.1 étant sélectionné aléatoirement,

C1.3 l'atome sélectionné dans l'étape C1.1 étant dévié à l'aide du vecteur de l'étape C1.2 à partir de sa position dans la géométrie de départ optimisée de telle sorte qu'un stade préliminaire à l'état de transition de la réaction chimique est obtenu,

C2 optimisation géométrique du stade préliminaire à l'état de transition au moyen de la méthode de chimie quantique et sous la condition limite selon laquelle ladite au moins une liaison de l'étape A2 présente la longueur qui a été déterminée dans l'étape C1.3,

C3 détermination de la norme de gradient C3 pour le stade préliminaire à l'état de transition de l'étape C2, la norme de gradient étant obtenue par la première dérivée d'une fonction E = f(x) au moyen de la méthode de chimie quantique, avec E = énergie totale du stade préliminaire à l'état de transition et x = les coordonnées de noyau de la molécule dans le stade préliminaire à l'état de transition,

C4.1 lorsque la norme de gradient C3 V répond à $0 \leq V \leq 0{,}07 \, E_h \, a_0^{-1}$, alors poursuite du procédé par l'étape D1 ou C4.2 lorsque la norme de gradient C3 V répond à $> 0{,}07 \, E_h \, a_0^{-1}$, alors répétition des étapes C1 à C3 jusqu'à ce qu'une norme de gradient C3 répondant à $0 \leq \nabla \leq 0{,}07 \, E_h \, a_0^{-1}$ soit obtenue

(a) pour le cas où les étapes C1 à C3 ont été effectuées une fois, dans l'étape C1, la géométrie du stade préliminaire à l'état de transition étant modifiée lorsque la valeur de sa norme de gradient C3 est inférieure à la valeur de la norme de gradient B3 de la géométrie de départ optimisée ou

(b) pour le cas où les étapes C1 à C3 ont été effectuées plus d'une fois, dans l'étape C1, la géométrie de la géométrie de départ optimisée ou du stade préliminaire respectif à l'état de transition, provenant des répétitions précédentes, qui présente la valeur la plus basse pour la norme de gradient C3 ou B3 par rapport à toutes les normes de gradient C3 et B3 obtenues jusqu'à présent, étant modifiée,

D détermination de l'état de transition

D1 relaxation du stade préliminaire de l'étape C4.1 ou du stade préliminaire de l'étape B4.1 au moyen de la méthode de chimie quantique et d'un algorithme de pseudo-Newton-Raphson, de telle sorte que l'état de transition est obtenu,

D2 le cas échéant détermination d'un état d'équilibre par déviation de l'état de transition, de telle sorte qu'un état de transition dévié est obtenu et relaxation de l'état de transition dévié au moyen de la méthode de chimie quantique, de telle sorte qu'un état d'équilibre est obtenu.

2. Procédé selon la revendication 1, **caractérisé en ce que** la méthode de chimie quantique des étapes B2, B3, C2, C3, D1 et D2 est une méthode semi-empirique, une méthode de la théorie de la fonctionnelle de la densité ou une approche de l'équation de Schrödinger, en particulier, la méthode de chimie quantique des étapes B2, B3, C2, C3, D1 et D2 est une méthode de la théorie de la fonctionnelle de la densité.

3. Procédé selon la revendication 1 ou 2, **caractérisé en ce que** la réaction chimique est une synthèse choisie dans le groupe constitué par les synthèses de polymères, en particulier les synthèses de polyuréthanes, les synthèses de monomères pour des réactions de polymérisation, des produits chimiques de base nécessaires dans l'industrie, des additifs, des tensioactifs et des substances actives pharmacologiques.

4. Procédé selon l'une des revendications précédentes, **caractérisé en ce que**, dans l'étape A1, au moins deux molécules I et II sont mises à disposition et, dans l'étape A2, en variante ou en plus de ladite au moins une liaison, on peut sélectionner également au moins une distance entre au moins un atome de la molécule I et au moins un atome de la molécule II ainsi que la longueur de ladite au moins une distance, la longueur de la distance valant en particulier au plus 230 pm.

5. Procédé selon la revendication 4, **caractérisé en ce que** la molécule I est un catalyseur pour la réaction chimique, en particulier pour une synthèse de polymères, et la molécule II est un produit de départ de la réaction chimique.

6. Procédé selon la revendication 4 ou 5, **caractérisé en ce que** la molécule I présente une dimension $\leq 100$ atomes et la molécule II présente une dimension $\leq 100$ atomes, de préférence la somme de atomes de la molécule I et de la molécule II représente $\leq 100$ atomes.

7. Procédé selon l'une des revendications 1 à 6, **caractérisé en ce que** l'information concernant l'état de transition déterminé selon l'étape D.1 et/ou l'état d'équilibre déterminé selon l'étape D.2 est communiquée à un utilisateur.

8. Procédé selon l'une des revendications 1 à 6, **caractérisé en ce que** l'information concernant l'état de transition déterminé selon l'étape D.1 et/ou l'état d'équilibre déterminé selon l'étape D.2 est reçue par un utilisateur.

9. Procédé selon la revendication 5 ou 6, **caractérisé en ce que** la molécule I est synthétisée après l'étape D.1 et/ou après l'étape D.2.

10. Procédé selon la revendication 5 ou 6, **caractérisé en ce qu'**après l'étape D.1 et/ou après l'étape D.2, une réaction chimique est effectuée avec la molécule I en tant que catalyseur.

11. Procédé selon la revendication 5, 6 ou 10, **caractérisé en ce qu'**après l'étape D.1 et/ou après l'étape D.2, une réaction chimique est effectuée avec la molécule II en tant que partenaire de réaction.

12. Système de traitement de données comprenant des moyens d'exécution d'un procédé comprenant les étapes :

A génération d'une géométrie de départ
A1 mise à disposition de la représentation tridimensionnelle d'au moins une molécule dans l'état énergétique de base,
A2 sélection d'au moins une liaison de ladite au moins une molécule et sélection d'une longueur de la liaison, la longueur sélectionnée ne correspondant pas à la longueur de la liaison dans l'état énergétique de base de la molécule, de telle sorte qu'une géométrie de départ pour la réaction chimique est obtenue,
A3 représentation tridimensionnelle de la géométrie de départ en coordonnées cartésiennes et/ou internes,
B détermination d'une géométrie de départ optimisée
B1 définition d'un espace fonctionnel qui comprend ladite au moins une liaison de l'étape A2 et les atomes reliés par cette liaison,
B2 optimisation géométrique de la géométrique de départ sur la base de l'espace fonctionnel sélectionné dans l'étape B1 au moyen d'une méthode de chimie quantique et sous la condition limite selon laquelle la longueur sélectionnée dans l'étape A2 de ladite au moins une liaison est maintenue constante, de telle sorte qu'une géométrie de départ optimisée est obtenue,
B3 détermination de la norme de gradient B3 pour la géométrie de départ optimisée, la norme de gradient étant obtenue par la première dérivée d'une fonction E = f(x) au moyen de la méthode de chimie quantique, avec E = énergie totale de la géométrie de départ optimisée et x = les coordonnées de noyau de la molécule dans la géométrie de départ optimisée,
B4.1 lorsque la norme de gradient B3 V répond à $0 \leq \nabla \leq 0{,}07\ E_h\,a_0^{-1}$, alors classification de la géométrie de départ optimisée comme un stade préliminaire à l'état de transition de la réaction chimique et poursuite du procédé par l'étape D1 ou
B4.2 lorsque la norme de gradient B3 V répond à $> 0{,}07\ E_h\,a_0^{-1}$, alors détermination du stade préliminaire à l'état de transition de la réaction chimique partant de la géométrie de départ optimisée par un procédé comprenant les étapes suivantes :

C détermination du stade préliminaire à l'état de transition de la réaction chimique
C1 variation de la géométrie de départ optimisée à l'aide d'un algorithme de Monte-Carlo
C1.1 au moins un atome de l'espace fonctionnel sélectionné dans l'étape B1 étant sélectionné aléatoirement,
C1.2 un vecteur pour une déviation de l'atome sélectionné dans l'étape C1.1 étant sélectionné aléatoirement,
C1.3 l'atome sélectionné dans l'étape C1.1 étant dévié à l'aide du vecteur de l'étape C1.2 à partir de sa position dans la géométrie de départ optimisée de telle sorte qu'un stade préliminaire à l'état de transition de la réaction chimique est obtenu,
C2 optimisation géométrique du stade préliminaire à l'état de transition au moyen de la méthode de chimie quantique et sous la condition limite selon laquelle ladite au moins une liaison de l'étape A2 présente la longueur qui a été déterminée dans l'étape C1.3,
C3 détermination de la norme de gradient C3 pour le stade préliminaire à l'état de transition de l'étape C2, la norme de gradient étant obtenue par la première dérivée d'une fonction E = f(x) au moyen de la méthode de chimie quantique, avec E = énergie totale du stade préliminaire à l'état de transition et x = les coordonnées de noyau de la molécule dans le stade préliminaire à l'état de transition,

C4.1 lorsque la norme de gradient C3 V répond à $0 \leq \nabla \leq 0{,}07$ $E_h$ $a_0^{-1}$, alors poursuite du procédé par l'étape D1 ou C4.2 lorsque la norme de gradient C3 V répond à $> 0{,}07$ $E_h$ $a_0^{-1}$, alors répétition des étapes C1 à C3 jusqu'à ce qu'une norme de gradient C3 répondant à $0 \leq \nabla \leq 0{,}07$ $E_h$ $a_0^{-1}$ soit obtenue

> (a) pour le cas où les étapes C1 à C3 ont été effectuées une fois, dans l'étape C1, la géométrie du stade préliminaire à l'état de transition étant modifiée lorsque la valeur de sa norme de gradient C3 est inférieure à la valeur de la norme de gradient B3 de la géométrie de départ optimisée ou
> (b) pour le cas où les étapes C1 à C3 ont été effectuées plus d'une fois, dans l'étape C1, la géométrie de la géométrie de départ optimisée ou du stade préliminaire respectif à l'état de transition, provenant des répétitions précédentes, qui présente la valeur la plus basse pour la norme de gradient C3 ou B3 par rapport à toutes les normes de gradient C3 et B3 obtenues jusqu'à présent, étant modifiée,

D détermination de l'état de transition
D1 relaxation du stade préliminaire de l'étape C4.1 ou du stade préliminaire de l'étape B4.1 au moyen de la méthode de chimie quantique et d'un algorithme de pseudo-Newton-Raphson, de telle sorte que l'état de transition est obtenu,
D2 le cas échéant détermination d'un état d'équilibre par déviation de l'état de transition, de telle sorte qu'un état de transition dévié est obtenu et relaxation de l'état de transition dévié au moyen de la méthode de chimie quantique, de telle sorte qu'un état d'équilibre est obtenu.

**13.** Programme informatique comprenant des instructions qui permettent de mettre en œuvre les étapes suivantes d'un procédé lors de l'exécution du programme par un ordinateur :

A génération d'une géométrie de départ
A1 mise à disposition de la représentation tridimensionnelle d'au moins une molécule dans l'état énergétique de base,
A2 sélection d'au moins une liaison de ladite au moins une molécule et sélection d'une longueur de la liaison, la longueur sélectionnée ne correspondant pas à la longueur de la liaison dans l'état énergétique de base de la molécule, de telle sorte qu'une géométrie de départ pour la réaction chimique est obtenue,
A3 représentation tridimensionnelle de la géométrie de départ en coordonnées cartésiennes et/ou internes,
B détermination d'une géométrie de départ optimisée
B1 définition d'un espace fonctionnel qui comprend ladite au moins une liaison de l'étape A2 et les atomes reliés par cette liaison,
B2 optimisation géométrique de la géométrique de départ sur la base de l'espace fonctionnel sélectionné dans l'étape B1 au moyen d'une méthode de chimie quantique et sous la condition limite selon laquelle la longueur sélectionnée dans l'étape A2 de ladite au moins une liaison est maintenue constante, de telle sorte qu'une géométrie de départ optimisée est obtenue,
B3 détermination de la norme de gradient B3 pour la géométrie de départ optimisée, la norme de gradient étant obtenue par la première dérivée d'une fonction E = f(x) au moyen de la méthode de chimie quantique, avec E = énergie totale de la géométrie de départ optimisée et x = les coordonnées de noyau de la molécule dans la géométrie de départ optimisée,
B4.1 lorsque la norme de gradient B3 V répond à $0 \leq \nabla \leq 0{,}07$ $E_h$ $a_0^{-1}$, alors classification de la géométrie de départ optimisée comme un stade préliminaire à l'état de transition de la réaction chimique et poursuite du procédé par l'étape D1 ou
B4.2 lorsque la norme de gradient B3 V répond à $> 0{,}07$ $E_h$ $a_0^{-1}$, alors détermination du stade préliminaire à l'état de transition de la réaction chimique partant de la géométrie de départ optimisée par un procédé comprenant les étapes suivantes :

C détermination du stade préliminaire à l'état de transition de la réaction chimique
C1 variation de la géométrie de départ optimisée à l'aide d'un algorithme de Monte-Carlo
C1.1 au moins un atome de l'espace fonctionnel sélectionné dans l'étape B1 étant sélectionné aléatoirement,
C1.2 un vecteur pour une déviation de l'atome sélectionné dans l'étape C1.1 étant sélectionné aléatoirement,
C1.3 l'atome sélectionné dans l'étape C1.1 étant dévié à l'aide du vecteur de l'étape C1.2 à partir de sa position dans la géométrie de départ optimisée de telle sorte qu'un stade préliminaire à l'état de transition de la réaction chimique est obtenu,

C2 optimisation géométrique du stade préliminaire à l'état de transition au moyen de la méthode de chimie quantique et sous la condition limite selon laquelle ladite au moins une liaison de l'étape A2 présente la longueur qui a été déterminée dans l'étape C1.3,

C3 détermination de la norme de gradient C3 pour le stade préliminaire à l'état de transition de l'étape C2, la norme de gradient étant obtenue par la première dérivée d'une fonction E = f(x) au moyen de la méthode de chimie quantique, avec E = énergie totale du stade préliminaire à l'état de transition et x = les coordonnées de noyau de la molécule dans le stade préliminaire à l'état de transition,

C4.1 lorsque la norme de gradient C3 V répond à $0 \leq \nabla \leq 0,07\ E_h\ a_0^{-1}$, alors poursuite du procédé par l'étape D1 ou C4.2 lorsque la norme de gradient C3 V répond à $> 0,07\ E_h\ a_0^{-1}$, alors répétition des étapes C1 à C3 jusqu'à ce qu'une norme de gradient C3 répondant à $0 \leq \nabla \leq 0,07\ E_h\ a_0^{-1}$ soit obtenue

> (a) pour le cas où les étapes C1 à C3 ont été effectuées une fois, dans l'étape C1, la géométrie du stade préliminaire à l'état de transition étant modifiée lorsque la valeur de sa norme de gradient C3 est inférieure à la valeur de la norme de gradient B3 de la géométrie de départ optimisée ou
> (b) pour le cas où les étapes C1 à C3 ont été effectuées plus d'une fois, dans l'étape C1, la géométrie de la géométrie de départ optimisée ou du stade préliminaire respectif à l'état de transition, provenant des répétitions précédentes, qui présente la valeur la plus basse pour la norme de gradient C3 ou B3 par rapport à toutes les normes de gradient C3 et B3 obtenues jusqu'à présent, étant modifiée,

D détermination de l'état de transition

D1 relaxation du stade préliminaire de l'étape C4.1 ou du stade préliminaire de l'étape B4.1 au moyen de la méthode de chimie quantique et d'un algorithme de pseudo-Newton-Raphson, de telle sorte que l'état de transition est obtenu,

D2 le cas échéant détermination d'un état d'équilibre par déviation de l'état de transition, de telle sorte qu'un état de transition dévié est obtenu et relaxation de l'état de transition dévié au moyen de la méthode de chimie quantique, de telle sorte qu'un état d'équilibre est obtenu.

14. Support d'enregistrement lisible par un ordinateur, comprenant des instructions qui permettent de mettre en œuvre les étapes suivantes d'un procédé lors de l'exécution par un ordinateur :

> A génération d'une géométrie de départ
> A1 mise à disposition de la représentation tridimensionnelle d'au moins une molécule dans l'état énergétique de base,
> A2 sélection d'au moins une liaison de ladite au moins une molécule et sélection d'une longueur de la liaison, la longueur sélectionnée ne correspondant pas à la longueur de la liaison dans l'état énergétique de base de la molécule, de telle sorte qu'une géométrie de départ pour la réaction chimique est obtenue,
> A3 représentation tridimensionnelle de la géométrie de départ en coordonnées cartésiennes et/ou internes,
> B détermination d'une géométrie de départ optimisée B1 définition d'un espace fonctionnel qui comprend ladite au moins une liaison de l'étape A2 et les atomes reliés par cette liaison,
> B2 optimisation géométrique de la géométrique de départ sur la base de l'espace fonctionnel sélectionné dans l'étape B1 au moyen d'une méthode de chimie quantique et
> sous la condition limite selon laquelle la longueur sélectionnée dans l'étape A2 de ladite au moins une liaison est maintenue constante, de telle sorte qu'une géométrie de départ optimisée est obtenue,
> B3 détermination de la norme de gradient B3 pour la géométrie de départ optimisée, la norme de gradient étant obtenue par la première dérivée d'une fonction E = f(x) au moyen de la méthode de chimie quantique, avec E = énergie totale de la géométrie de départ optimisée et x = les coordonnées de noyau de la molécule dans la géométrie de départ optimisée,
> B4.1 lorsque la norme de gradient B3 V répond à $0 \leq \nabla \leq 0,07\ E_h\ a_0^{-1}$, alors classification de la géométrie de départ optimisée comme un stade préliminaire à l'état de transition de la réaction chimique et poursuite du procédé par l'étape D1 ou
> B4.2 lorsque la norme de gradient B3 V répond à $> 0,07\ E_h\ a_0^{-1}$, alors détermination du stade préliminaire à l'état de transition de la réaction chimique partant de la géométrie de départ optimisée par un procédé comprenant les étapes suivantes :

> > C détermination du stade préliminaire à l'état de transition de la réaction chimique
> > C1 variation de la géométrie de départ optimisée à l'aide d'un algorithme de Monte-Carlo

C1.1 au moins un atome de l'espace fonctionnel sélectionné dans l'étape B1 étant sélectionné aléatoirement,

C1.2 un vecteur pour une déviation de l'atome sélectionné dans l'étape C1.1 étant sélectionné aléatoirement,

C1.3 l'atome sélectionné dans l'étape C1.1 étant dévié à l'aide du vecteur de l'étape C1.2 à partir de sa position dans la géométrie de départ optimisée de telle sorte qu'un stade préliminaire à l'état de transition de la réaction chimique est obtenu,

C2 optimisation géométrique du stade préliminaire à l'état de transition au moyen de la méthode de chimie quantique et sous la condition limite selon laquelle ladite au moins une liaison de l'étape A2 présente la longueur qui a été déterminée dans l'étape C1.3,

C3 détermination de la norme de gradient C3 pour le stade préliminaire à l'état de transition de l'étape C2, la norme de gradient étant obtenue par la première dérivée d'une fonction $E = f(x)$ au moyen de la méthode de chimie quantique, avec $E$ = énergie totale du stade préliminaire à l'état de transition et $x$ = les coordonnées de noyau de la molécule dans le stade préliminaire à l'état de transition,

C4.1 lorsque la norme de gradient C3 V répond à $0 \leq \nabla \leq 0,07\ E_h\ a_0^{-1}$, alors poursuite du procédé par l'étape D1 ou C4.2 lorsque la norme de gradient C3 V répond à $> 0,07\ E_h\ a_0^{-1}$, alors répétition des étapes C1 à C3 jusqu'à ce qu'une norme de gradient C3 répondant à $0 \leq \nabla \leq 0,07\ E_h\ a_0^{-1}$ soit obtenue

> (a) pour le cas où les étapes C1 à C3 ont été effectuées une fois, dans l'étape C1, la géométrie du stade préliminaire à l'état de transition étant modifiée lorsque la valeur de sa norme de gradient C3 est inférieure à la valeur de la norme de gradient B3 de la géométrie de départ optimisée ou
> (b) pour le cas où les étapes C1 à C3 ont été effectuées plus d'une fois, dans l'étape C1, la géométrie de la géométrie de départ optimisée ou du stade préliminaire respectif à l'état de transition, provenant des répétitions précédentes, qui présente la valeur la plus basse pour la norme de gradient C3 ou B3 par rapport à toutes les normes de gradient C3 et B3 obtenues jusqu'à présent, étant modifiée,

D détermination de l'état de transition

D1 relaxation du stade préliminaire de l'étape C4.1 ou du stade préliminaire de l'étape B4.1 au moyen de la méthode de chimie quantique et d'un algorithme de pseudo-Newton-Raphson, de telle sorte que l'état de transition est obtenu,

D2 le cas échéant détermination d'un état d'équilibre par déviation de l'état de transition, de telle sorte qu'un état de transition dévié est obtenu et relaxation de l'état de transition dévié au moyen de la méthode de chimie quantique, de telle sorte qu'un état d'équilibre est obtenu.

15. Utilisation d'un programme informatique selon la revendication 13 ou d'un support d'enregistrement lisible par un ordinateur selon la revendication 14 pour l'évaluation d'états de transition d'une réaction chimique, en particulier d'une synthèse de polymères.

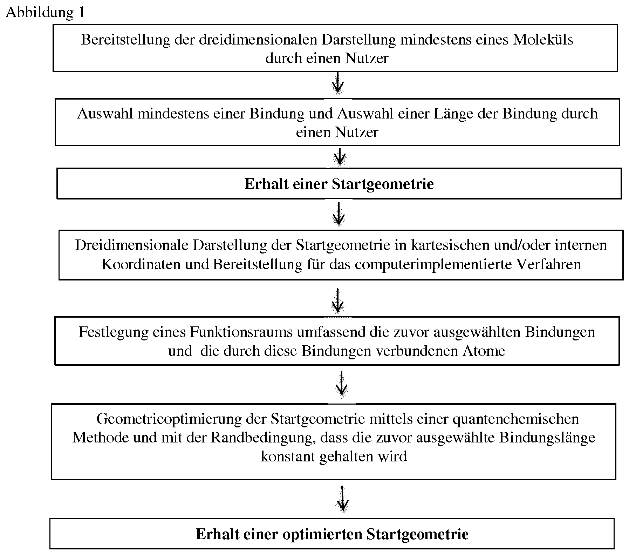

Abbildung 1

| Bereitstellung der dreidimensionalen Darstellung mindestens eines Moleküls durch einen Nutzer |

↓

| Auswahl mindestens einer Bindung und Auswahl einer Länge der Bindung durch einen Nutzer |

↓

| **Erhalt einer Startgeometrie** |

↓

| Dreidimensionale Darstellung der Startgeometrie in kartesischen und/oder internen Koordinaten und Bereitstellung für das computerimplementierte Verfahren |

↓

| Festlegung eines Funktionsraums umfassend die zuvor ausgewählten Bindungen und die durch diese Bindungen verbundenen Atome |

↓

| Geometrieoptimierung der Startgeometrie mittels einer quantenchemischen Methode und mit der Randbedingung, dass die zuvor ausgewählte Bindungslänge konstant gehalten wird |

↓

| **Erhalt einer optimierten Startgeometrie** |

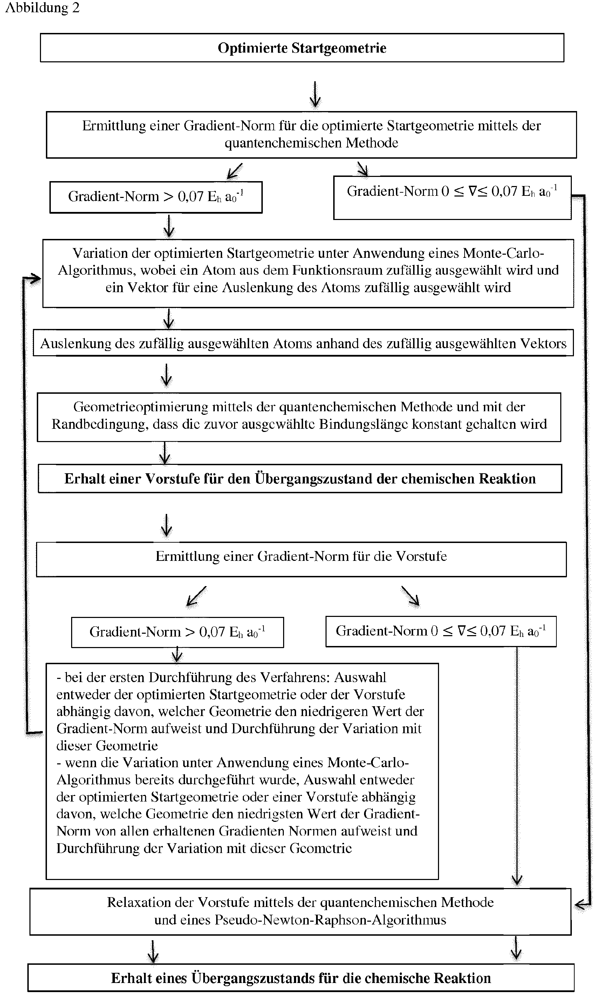

Abbildung 2

**Optimierte Startgeometrie**

Ermittlung einer Gradient-Norm für die optimierte Startgeometrie mittels der quantenchemischen Methode

Gradient-Norm $> 0{,}07\ E_h\ a_0^{-1}$

Gradient-Norm $0 \leq \nabla \leq 0{,}07\ E_h\ a_0^{-1}$

Variation der optimierten Startgeometrie unter Anwendung eines Monte-Carlo-Algorithmus, wobei ein Atom aus dem Funktionsraum zufällig ausgewählt wird und ein Vektor für eine Auslenkung des Atoms zufällig ausgewählt wird

Auslenkung des zufällig ausgewählten Atoms anhand des zufällig ausgewählten Vektors

Geometrieoptimierung mittels der quantenchemischen Methode und mit der Randbedingung, dass die zuvor ausgewählte Bindungslänge konstant gehalten wird

**Erhalt einer Vorstufe für den Übergangszustand der chemischen Reaktion**

Ermittlung einer Gradient-Norm für die Vorstufe

Gradient-Norm $> 0{,}07\ E_h\ a_0^{-1}$

Gradient-Norm $0 \leq \nabla \leq 0{,}07\ E_h\ a_0^{-1}$

- bei der ersten Durchführung des Verfahrens: Auswahl entweder der optimierten Startgeometrie oder der Vorstufe abhängig davon, welcher Geometrie den niedrigeren Wert der Gradient-Norm aufweist und Durchführung der Variation mit dieser Geometrie
- wenn die Variation unter Anwendung eines Monte-Carlo-Algorithmus bereits durchgeführt wurde, Auswahl entweder der optimierten Startgeometrie oder einer Vorstufe abhängig davon, welche Geometrie den niedrigsten Wert der Gradient-Norm von allen erhaltenen Gradienten Normen aufweist und Durchführung der Variation mit dieser Geometrie

Relaxation der Vorstufe mittels der quantenchemischen Methode und eines Pseudo-Newton-Raphson-Algorithmus

**Erhalt eines Übergangszustands für die chemische Reaktion**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Nicht-Patentliteratur**

- **LIN et al.** A flexible transition state searching method for atmospheric reaction systems. *Chemical Physics*, 2015, vol. 450-451, 21-31
- **E. MARTÍNEZ-NÚÑEZ et al.** An automated transition state search using classical trajectories initialized at multiple minima. *Phys Chem Chem Phys*, 14 June 2015, vol. 17 (22), 14912-21
- tsscds2018: A code for automated discovery of chemical reaction mechanisms and solving the kinetics. *J. Comp.Chem.*, 24 September 2018, vol. 39 (23), 1922-1930
- **JACOBSON et al.** Automated Transition State Search and Its Application to Diverse Types of Organic Reactions. *J. Chem. Theory Comput.*, vol. 13 (11), 5780-5797
- **HU et al.** A gradient-directed Monte Carlo method for global optimization in a discrete space: Application to protein sequence design and folding. *J Chem Phys.*, 21 October 2009, vol. 131 (15), 154117